# EUROPÄISCHE PATENTSCHRIFT

(11) **EP 3 362 119 B1**
(45) Veröffentlichungstag und Bekanntmachung des Hinweises auf die Patenterteilung: **25.03.2020**
(21) Anmeldenummer: 16781481.3
(22) Anmeldetag: 14.10.2016
(51) Int. Cl.: A61M 1/16, A61B 5/145, A61M 1/10

(54) **MEMBRANKATHETER**
MEMBRANE CATHETER
CATHETER A MEMBRANE

(30) Priorität: 14.10.2015 EP 15189777
(43) Veröffentlichungstag der Anmeldung: 22.08.2018
(73) Patentinhaber: CCore Technology GmbH, 1040 Wien (AT)
(72) Erfinder: NEUDL, Susanna, 1190 Wien (AT); ULLRICH, Roman, 1180 Wien (AT); KRENN, Claus-Georg, 2340 Mödling (AT); GFÖHLER, Margit, 1230 Wien (AT); JANECZEK, Christoph, 2603 Felixdorf (AT)
(74) Vertreter: Sonn & Partner Patentanwälte
(86) Internationale Anmeldenummer: PCT/EP2016/074776
(87) Internationale Veröffentlichungsnummer: WO 2017/064285

(56) Entgegenhaltungen:
- WO-A1-2008/046630
- WO-A2-2004/016300
- GB-A- 2 505 068
- US-A- 4 631 053
- US-A1- 2010 258 116
- US-A1- 2013 053 623

## Beschreibung

Die Erfindung betrifft eine Vorrichtung zum Austausch von Substanzen aus dem Blut oder in das Blut.

Das Anwendungsgebiet der Erfindung ist ganz allgemein die Untersuchung oder Behandlung des menschlichen oder tierischen Körpers. Im Einzelnen wird dabei der Transport bestimmter Substanzen entweder aus dem Blut des Körpers nach außerhalb des Körpers oder - umgekehrt - von außerhalb des Körpers in das Blut im Körper ermöglicht. In dieses allgemeine Anwendungsgebiet fallen insbesondere Diagnoseverfahren, welche auf dem Nachweis bestimmter Substanzen im Blut oder auch auf quantitativen Messungen bestimmter Substanzen im Blut beruhen. Weiters umfasst das Anwendungsgebiet der Erfindung auch Funktionen, welche die Tätigkeit von Organen im Körper, insbesondere der Lunge, der Nieren oder der Leber unterstützen oder ersetzen. Schließlich kann die Erfindung auch bei der Verabreichung von nicht körpereigenen Substanzen, insbesondere Medikamenten, Anwendung finden. Besonders vielversprechende Anwendungsgebiete betreffen eine über einen längeren Zeitraum andauernde Unterstützung der Lungenfunktion oder der Nierenfunktion (Continuous Renal Replacement Therapy, CRRT).

Unter das obige Anwendungsgebiet fällt beispielsweise auch eine extrakorporale Blutreinigung (Extracorporeal blood purification, ECBP). Diese betrifft eine Entfernung endogener oder exogener Toxine oder gelöster Stoffe aus dem Blut und umfasst verschiedene Therapien, wie Hämodialyse, Hämofiltration oder Hämodiafiltration. Der Leitgedanke ist die Entnahme von Blut aus dem Patienten, Entfernung der Substanz in einer externen Filtereinheit ("Reinigung") und Rückführung von gereinigtem Blut über einen venösen Zugang.

Die GB 2 004 092 A zeigt eine Vorrichtung zur Messung des Blutzuckergehalts mittels eines intravaskulären Membran-Katheters. Dabei wird eine Messflüssigkeit in einem Kreislauf an einer Membran, welche die Außenseite des Katheters bildet, vorbei geführt und aus dem Körper geleitet, wo die Flüssigkeit zunächst mit Sauerstoff angereichert wird. Anschließend wird die Messflüssigkeit einem Enzymreaktor zugeführt und der Blutzuckergehalt aus dem nach dem Reaktor verbleibenden Sauerstoff-Partialdruck ermittelt. Die Oberfläche der Membran ist bei diesem Aufbau durch die Abmessungen des Katheters begrenzt, sodass nur ein vergleichsweise geringer Anteil des Bluts in Kontakt mit der Membran kommt und an dem Austausch mit der Messflüssigkeit teilnimmt.

Die WO 2008/046630 A1 zeigt Vorrichtungen zur kontinuierlichen Überwachung der Konzentration einer Substanz im Blut mittels Mikrodialyse. Dabei kann eine von Blut umgebene und mit Perfusat durchströmte Rohrmembran verwendet werden. Eine solche Rohrmembran weist naturgemäß keinen Bluteinlass oder -auslass auf. Alternativ zur Rohrmembran ist eine planare Membran beschrieben, wobei - offensichtlich extrakorporeal - Blut und Trägermedium durch jeweils auf gegenüber liegenden Seiten der planaren Membran vorgesehene, in Mäandern verlaufende Leitungen gepumpt wird.

Weiters ist es auf dem Gebiet der Erfindung bereits bekannt, die Lungenfunktion eines Patienten mit ECMO (Extra Corporeal Membrane Oxygenation) oder ECCO2R (Extra Corporeal CO₂ Removal) Systemen zu unterstützen. Derartige Systeme umfassen in der Regel Kanülen, eine Kreiselpumpe, ein Schlauchsystem, einen Membranoxygenator, Sicherheitssysteme und einen Wärmetauscher und sind zuverlässig und einfach zu handhaben. Sie weisen allerdings mehrere Limitationen für eine weite Verbreitung und häufige Anwendung auf - sie sind sehr teuer, sehr groß und machen Patienten immobil, das heißt der Patient muss während der Behandlung ruhiggestellt (sediert) werden, da durch Bewegungen eine Dislokation der Kanülen droht. Außerdem ist bei den gängigen Systemen eine Herausleitung des Blutes aus dem Körper über einen oder mehrere Katheter und eine Rückführung des mit Sauerstoff angereicherten und von CO₂ befreiten Blutes in den Körper erforderlich.

Viele Patienten mit akuter und chronischer respiratorischer CO₂ Erhöhung (Hyperkapnia) würden daher von der Verfügbarkeit einer verkleinerten, mobilen Form der ECCO2R - mit allen zu erwartenden Vorteilen - profitieren, da sie dadurch früher mobilisiert werden können, keine langdauernde oder weitere Sedierung benötigen und diese Therapie auch bereits in der Frühphase der Erkrankung eingesetzt werden könnte. In extenso ist eine völlige Vermeidung maschineller Beatmung denkbar, da ein Großteil der Patienten erst aufgrund der respiratorischen Erschöpfung und der Hyperkapnie, infolge erhöhter Atemarbeit, beatmungspflichtig werden.

Bereits zur Marktreife entwickelte intravaskuläre Membranoxygenatoren wurden in der Vergangenheit klinisch erprobt und zur Verwendung an Patienten zugelassen. Diese Techniken hatten primär das Ziel, den Mangel an Sauerstoff im Blut durch intravaskuläre Membranoxygenierung zu verbessern oder ein genügendes Sauerstoffangebot zu gewährleisten. Um dieses Ziel zu erreichen, bedarf es einer großen Kontaktoberfläche zwischen Blut und Gasphase und einer Zufuhr von Gasen in den Katheter im Gefäßsystem, was potentiell die Gefahr einer Gasembolie massiv erhöht. Das Problem der notwendig großen Mindestoberfläche wurde mit der Einführung von entfaltbaren Netzen aus Hohlfasern, die extrakorporal mit hohen Gasflüssen durchspült wurden, gelöst. Diese Technik hat mehrere Limitationen, darunter ein niedriger Gastransfer für Sauerstoff (O₂), eine hohe Thrombogenität des turbulent durchströmten Fasersystems, die Anfälligkeit der Hohlfasern zu brechen und die zwingende Größe des Katheters mit allen damit verbundenen Verletzungsrisiken. Aus diesen Gründen konnte sich diese Technologie in der Klinik nicht durchsetzen.

Intravaskuläre Membranoxygenatoren sind in unterschiedlichen Ausführungsvarianten aus der Patentliteratur bekannt. So offenbart beispielsweise die WO 2004/016300 A2 einen als Katheter ausgeführten intravenösen Oxygenator zur Sauerstoffanreicherung von Blut mit einem Faserbündel, wobei die Fasern jeweils mit einem ersten Anschluss an eine Gaszufuhr und mit einem zweiten Anschluss an einen Gasabzug angeschlossen sind. Das Faserbündel ist tordiert durch eine relative Verdrehung des ersten Anschlusses der Fasern gegenüber dem zweiten Anschluss der Fasern im Betrieb um die Längsachse des Oxygenators. Die Fasern verlaufen daher über die gesamte Länge des Faserbündels als durchgehende Gasleitungen. Im Betrieb des Oxygenators wird Sauerstoff zugeführt, welcher über den ersten Anschluss in die Fasern strömt, an deren Oberfläche ein diffuser Gasaustausch mit Blut stattfindet. Dabei kommt es zu einer Anreicherung des Bluts mit Sauerstoff bei gleichzeitiger Entfernung von CO₂. Am zweiten Anschluss befindet sich in den Fasern daher ein Gasgemisch aus Sauerstoff und Kohlendioxid, welches durch eine Abzugkammer in einem Schlauch und durch diesen aus dem Körper des Patienten herausströmt. Das in den Oxygenator strömende Blut durchströmt das tordierte Faserbündel und gelangt zu einer Pumpe. Das Blut wird dort in Fließrichtung der Vene befördert und verlässt den Oxygenator durch einen Auslass. Der Druckabfall des Blutes wird daher durch die Pumpe ausgeglichen, sodass der Druck am Auslass wieder den physiologischen Druck aufweist. Ein effektiver Austausch von Sauerstoff gegen Kohlendioxid erfordert eine sehr große, in einem Katheter kaum realisierbare Membranoberfläche der Hohlfasern, was im Gegenzug die Gefahr des Entstehens einer Gasembolie weiter steigert.

Die US 2010/0258116 A1 befasst sich mit unterschiedlichen Verfahren zum Austausch von Kohlendioxid und insbesondere mit Blutoxygenatoren. Allerdings wird darin nicht näher auf bestimmte Austauschvorrichtungen eingegangen und es werden sowohl extrakorporale Austauscheinrichtungen als auch intravaskuläre Katheter erwähnt. Die Struktur der Katheter ist nicht genau beschrieben. Insbesondere ist kein Kathetehr mit einem Bluteinlass und Blutauslass gezeigt. Außerdem ist im Bezug auf Katheter ausschließlich die Zuführung von Gasen als Austsuchmedium erwähnt.

Die US 4,631,053 A offenbart einen intravaskulären Membranoxygenator. Als Austauschmedium ist ausschließlich die Verwendung von - unter Standardbedingungen gasförmigem - Sauerstoff offenbart. Ein Trägermedium wird offenbar nicht verwendet, da nur eine Zuführleitung für den Sauerstoff und keine Rückführung für ein etwaiges Trägermedium gezeigt ist.

In GB 2 505 068 A ist ein extrakorporaler Antrieb gezeigt, welcher über eine Welle mit einer Katheterpumpe zur Unterstützung der Herzfunktion verbunden ist. Die offenbarte Antriebseinheit ist aufgrund ihrer Größe offensichtlich ungeeignet, um als Teil eines intravaskulären Katheters verwendet zu werden.

Die in US 2013/053623 A1 gezeigte Pumpe dient der Unterstützung der Herzfunktion und betrifft somit ein gänzlich anderes Anwendungsgebiet als die vorliegende Vorrichtung. Die gezeigte Pumpe umfasst naturgemäß keine Membran im Sinn der erfindungsgemäßen Vorrichtung, d.h. zum Austausch von Substanzen.

Ausgehend von den genannten Nachteilen der bekannten Vorrichtungen und Verfahren ist es Aufgabe der Erfindung, eine Vorrichtung zu schaffen, mit der bzw. dem effizient Substanzen aus dem Blut oder in das Blut transportiert werden können. Weiters soll diese Vorrichtung den Kriterien minimalinvasiver Eingriffe genügen und den Patienten und dessen Blutkreislauf wenig beeinträchtigen. Insbesondere sollen die Nachteile eines extrakorporealen Kreislaufes und unnötige Oberflächenkontakte des Bluts verringert werden. Weiters soll die Entfernung von toxischen Stoffen aus dem Blut gegenüber gegenwärtig angewendeten Verfahren verbessert werden. Beispielsweise soll mit dem erfindungsgemäßen System die Gefahr einer Gasembolie reduziert und zugleich die Leistungsfähigkeit der Austauschvorrichtung, insbesondere hinsichtlich des Verhältnisses zwischen der Austauschrate der Membran und der dafür nötigen Größe des Katheters, verbessert werden.

Die vorliegende Erfindung betrifft daher eine Vorrichtung gemäß Anspruch 1.

Weiters wird ein Verfahren zur Entnahme zumindest einer Substanz aus venösem Blut zu Diagnosezwecken unter Verwendung einer der genannten Vorrichtungen offenbart, wobei die zu entnehmende Substanz der durch die Membran des Katheters der Vorrichtung auszutauschenden Substanz entspricht.

Außerdem wird ein Verfahren zur Behandlung eines menschlichen oder tierischen Körpers durch einen Austausch zumindest einer Substanz aus dem Blut oder in das Blut des Körpers unter Verwendung einer der genannten Vorrichtungen offenbart.

Erfindungsgemäß ist daher vorgesehen, dass das Trägermedium eine Trägerflüssigkeit ist, in welcher die auszutauschende Substanz löslich ist. D.h. das Trägermedium befindet sich im Betrieb (bei Standardbedingungen) im flüssigen Aggregatzustand. Aufgrund der Verwendung einer Flüssigkeit als Trägermedium ist der Ab- und Zutransport von Substanzen wesentlich kontrollierter und effizienter durchführbar; beispielsweise wird dadurch die Gefahr einer Gasembolie deutlich reduziert. Außerdem besteht nur eine geringe Gefahr der Entstehung von Thrombosen. Da das Blut nicht aus dem Patienten heraus geleitet wird, entfällt die Notwendigkeit einer systemischen Antikoagulation mit den damit verbundenen Blutungskomplikationen. Davon abgesehen kann durch die Verwendung geeigneter Trägerflüssigkeiten und rascherer/höherer Zirkulation ein im Allgemeinen höheren Austausch der auszutauschenden Substanz durch die Membran, d.h. durch eine für Flüssigkeiten geeignete Membran (kurz "Flüssigkeitsmembran"), erzielt und die nötige Membranoberfläche somit verringert werden, was zu einer verringerten Druckdifferenz im Katheter führt. Falls der Katheter eine Fördereinrichtung umfasst, ist die zum Angleichen der geringeren Druckdifferenz benötigte Förderleistung der Fördereinrichtung dementsprechend ebenfalls verringert, was im Allgemeinen eine Verkleinerung der Fördereinrichtung und somit des Katheters insgesamt erlaubt. Im Falle eines Katheters ohne Fördereinrichtung kann aufgrund der vergleichsweise geringeren Druckdifferenz eine Verkleinerung des Querschnitts des Katheters vorgenommen werden. Durch die somit mit der Erfindung einhergehende Möglichkeit der Verkleinerung des gesamten Systems wird die Applikation erleichtert und dadurch letztlich die Gefahr von Komplikationen verringert. Das gesamte System kann unter Verwendung von bereits auf dem Markt befindlichen Komponenten so dimensioniert werden, dass es tragbar ist und der extrakorporale Kreislauf mit oder an der Konsole sogar in eine Tasche passt.

Aufgrund der Verwendung einer Trägerflüssigkeit als Trägermedium ist die Membran der Vorrichtung eine für Flüssigkeiten geeignete Membran, d.h. zum Austausch von Substanzen zwischen zwei Flüssigkeiten konfiguriert und eingerichtet. Auch wenn mit einer solchen Membran auch in gewissem Ausmaß ein Austausch von Substanzen zwischen einer Flüssigkeit und einem Gas erzielt werden könnte oder kann, ergibt sich aus der Konfiguration der Membran insgesamt, vor allem aus der vorgesehenen Kontaktfläche und/oder aus der mechanischen Stabilität, dass sie nicht für die Verwendung mit einem gasförmigen Trägermedium eingerichtet ist.

Die Fördereinrichtung umfasst eine Antriebseinheit zur Erzeugung eines Drehmoments und einen mit der Antriebseinheit zur Übertragung eines Drehmoments verbundenen Pumpenläufer. Das Drehmoment wird dabei vorzugsweise entlang einer Welle übertragen, deren Achse im Wesentlichen parallel zu einer Längserstreckungsachse des Katheters angeordnet ist. Der Pumpenläufer entspricht dabei dem Läufer einer Axialpumpe.

Eine einfache Steuerung der Fördereinrichtung, insbesondere der Drehzahl des Läufers, kann erzielt werden, wenn die Antriebseinheit einen Elektromotor umfasst. Die Zuverlässigkeit der Fördereinrichtung ist dabei im Wesentlichen durch die Zuverlässigkeit des Elektromotors begrenzt.

Alternativ kann die Antriebseinheit ein Turbinenelement umfassen, welches im Betrieb von dem Trägermedium umströmt wird. Bei einer solchen Fördereinrichtung wird ein Teil der Strömungsenergie des Trägermediums auf geförderte Blut übertragen. Im einfachsten und zuverlässigsten Fall ist das Turbinenelement direkt mit dem Pumpenläufer gekoppelt, sodass die beiden Elemente im Betrieb mit derselben Drehzahl laufen. Ein Antriebsverhältnis kann dabei über die Gestalt, d.h. die Fläche, Form und Anordnung, der jeweiligen Flügel bestimmt werden. Diese Art des Antriebs hat den zusätzlichen Vorteil, dass keine separate Energieversorgung, insbesondere kein elektrische Verbindung, der Antriebseinheit von außerhalb des Körpers erforderlich ist. Dadurch wird sowohl die Zuverlässigkeit als auch die Betriebssicherheit der Vorrichtung verbessert.

Im Zusammenhang mit der Antriebseinheit ist es günstig, dass der Pumpenläufer über eine Magnetkupplung mit der Antriebseinheit verbunden ist, wobei die Magnetkupplung zur Drehmomentübertragung entlang einer Drehachse zwei relativ zueinander drehbare Kupplungsteile mit jeweils einem Dauermagnet aufweist. Als Magnetkupplung kann beispielsweise eine Zentraldrehkupplung oder eine Stirndrehkupplung verwendet werden. Eine Magnetkupplung hat gegenüber einer durchgehenden mechanischen Verbindung, z.B. in Form einer durchgehenden Welle, den Vorteil, dass das übertragene Drehmoment begrenzt ist. Dadurch lassen sich insbesondere im Fehlerfall unvorhergesehene Zustände ausschließen; z.B. kann selbst bei Blockade eines Elements das jeweils gekoppelte andere Element mit gewissen Einschränkungen weiter beweglich bleiben. Wenn etwa der Antrieb des Pumpenläufers ausfällt oder blockiert, kann der Pumpenläufer nach Überwindung des von der Magnetkupplung maximal übertragenen Drehmoments annähernd frei laufen, wodurch im Vergleich zu einem blockierenden Pumpenläufer die Blutströmung einem geringeren Strömungswiderstand ausgesetzt ist, sodass die Gefahr von Komplikationen, z.B. von Thrombosen aufgrund der (an Fremdoberflächen aktivierten) Blutgerinnung reduziert wird. Darüber hinaus kann durch die Begrenzung des Drehmoments einer Schädigung der Pumpe aufgrund einer etwaigen Überlastung entgegen gewirkt werden.

Der Läufer oder Rotor der somit gebildeten Pumpe kann zudem vorzugsweise schwebend gelagert sein. Beispielsweise kann eine Lagerung entsprechend dem Herzunterstützungssystem INCOR der Firma "Berlin Heart" vorgesehen sein.

Um auch bei einer besonders kompakten Magnetkupplung ein gewünschtes Drehmoment übertragen zu können, umfasst einer der Kupplungsteile ein zumindest teilweise ferromagnetisches Umleitelement, welches mit dem Dauermagnet des Kupplungsteils drehfest verbunden ist, wobei ein Teil des Umleitelements radial außerhalb des Dauermagnets des anderen Kupplungsteils angeordnet ist. D.h. die Magnetkupplung umfasst zwei relativ zueinander drehbaren Kupplungsteile, wobei ein antriebsseitiger Kupplungsteil einen antriebsseitigen Dauermagnet aufweist und ein abtriebsseitiger Kupplungsteil einen dem antriebsseitigen Dauermagnet entlang der Drehachse gegenüberliegenden und im Abstand davon angeordneten abtriebsseitigen Dauermagnet aufweist, wobei einer der Kupplungsteile ein zumindest teilweise ferromagnetisches Umleitelement umfasst, welches mit dem Dauermagnet des Kupplungsteils drehfest verbunden ist, wobei ein Teil des Umleitelements radial außerhalb des gegenüberliegenden Dauermagnets angeordnet ist. Diese Bauform hat gegenüber herkömmlichen Zentraldrehkupplungen den Vorteil, dass sie einfacher und kostengünstiger herstellbar ist und eine insgesamt geringere Kupplungsfläche erfordert, da ein Teil des Drehmoment über die Stirnseite der Kupplungsteile übertragen wird. Gegenüber herkömmlichen Stirndrehkupplungen hat sie den Vorteil, dass zur Übertragung eines bestimmten Drehmoments geringere radiale Abmessungen erforderlich sind. Das Umleitelement kann dabei - vergleichbar dem äußeren Kupplungsteil einer Zentraldrehkupplung - topf- bzw. hohlzylinderförmig ausgebildet sein und den jeweils anderen Kupplungsteil umfangseitig umgeben, d.h. es erstreckt sich vorzugsweise radial außerhalb beider Dauermagnete. Dabei kann das Umleitelement beispielsweise als dünnwandiger Hohlzylinder ausgebildet sein, sodass bei gleichbleibenden Abmessungen das magnetisierte Volumen der Stirndrehkupplung weitestgehend erhalten bleibt und zugleich zwischen dem Umleitelement und dem im Abstand davon gegenüberliegenden Dauermagnet ein einer Zentraldrehkupplung vergleichbar großes übertragbares Drehmoment erzielbar ist. Die Magnetisierungsrichtung der Dauermagnete ist dabei vorzugsweise senkrecht auf die Drehachse ausgerichtet, d.h. die Magnetpole verlaufen in Umfangsrichtung von Süd nach Nord und liegen sich - jedenfalls bei einer zweipoligen Ausführung - bezüglich der Drehachse diametral gegenüber. Durch das Umleitelement werden radial von den Dauermagneten ausgehende magnetische Feldlinien gebündelt und die magnetische Kraft zwischen den Kupplungsteilen wegen des ferromagnetischen Materials des Umleitelements zusätzlich verstärkt. Durch die Verdichtung der magnetischen Feldlinien im ferromagnetischen Material wird die magnetische Kraft zur Übertragung des Drehmoments vergrößert. Aufgrund des im Vergleich zu Zentraldrehkupplungen bei gleichen Kupplungsabmessungen größeren Volumens der Dauermagneten kann vorteilhafter Weise eine geringere axiale Erstreckung und somit geringere radiale Querkräfte auf die Lager der Kupplungsteile erzielt werden.

Die Dauermagnete der Magnetkupplung können jeweils 2-, 4-, oder 6- polige Dauermagnete sein. Sie sind vorzugsweise zweipolig mit jeweils zwei halbzylinderförmigen Magnetpolen. Das Umleitelement weist zumindest eine diamagnetische Trennung auf, welche das Umleitelement in zumindest zwei ferromagnetische Abschnitte unterteilt und so einen magnetischen Kurzschluss vermeidet. Zusätzlich zur radial äußeren Anordnung kann sich das Umleitelement auch an eine dem gegenüberliegenden Dauermagnet abgewandten Rückseite des drehfest verbundenen Dauermagnets erstrecken. Alternativ oder zusätzlich kann das Umleitelement einen im Wesentlichen H-förmigen Längsschnitt aufweisen, mit einem senkrecht auf die Drehachse liegenden Quersteg und beidseitig topfförmigen Ausnehmungen, wobei in einer dieser Ausnehmungen ein Dauermagnet aufgenommen und drehfest verbunden ist. D.h. das Umleitelement kann einen hohlzylinderförmigen Mantel aufweisen und vorzugsweise mit einem auf im Wesentlichen halber Höhe des Mantels angeordneten Zwischenboden ausgebildet sein.

Eine besonders hohe Konzentration von Magnetfeldlinien im Umleitelement der Magnetkupplung kann erzielt werden, wenn an einer dem gegenüberliegenden Dauermagnet abgewandten Rückseite des drehfest mit dem Umleitelement verbundenen Dauermagnets ein diamagnetisches Abschirmelement angeordnet ist. Dadurch können außerhalb der Kupplungsteile verlaufende Feldlinien vermieden und damit verbundene Verluste reduziert werden.

Weiters hat es sich als günstig herausgestellt, wenn bei der Magnetkupplung an einer dem gegenüberliegenden Dauermagnet zugewandten Vorderseite des drehfest mit dem Umleitelement verbundenen Dauermagnets, insbesondere in einem um die Drehachse zentrierten Bereich, ein diamagnetisches Abschirmelement angeordnet ist, welches vorzugsweise umfangsseitig bzw. radial außenseitig an das Umleitelement anschließt. Mit einer derartigen Abschirmung kann eine Umlenkung des magnetischen Feldes in radial in größerem Abstand von der Drehachse befindliche Bereiche erzielt werden, sodass das bei gegebener Magnetkraft übertragene Drehmoment erhöht wird.

Um einen Übertritt des Trägermediums in das Blut zuverlässig zu vermeiden, ist es zudem günstig, wenn die beiden Kupplungsteile hermetisch getrennt sind. Eine solche hermetische Trennung kann etwa durch eine hermetische Wand zwischen den beiden Kupplungsteilen der Magnetkupplung erzielt werden, welche Wand weder magnetisch noch elektrisch leitend sein sollte. Insbesondere kann zumindest einer der Kupplungsteile in einem im Wesentlichen nichtmagnetischen und elektrisch nicht leitenden Gehäuse untergebracht sein, sodass Verluste aufgrund einer Ummagnetisierung des Gehäuses bzw. induzierte Wirbelströme im Gehäuse vermieden werden können.

Eine Anwendung, welche einen besonders hohen Nutzen aus den Vorteilen der Erfindung zieht, ist die Verwendung zum Abtransport von CO₂ aus dem Blut, d.h. wobei die auszutauschende Substanz CO₂ ist. Hierbei kann die Trägerflüssigkeit vorzugsweise eine Löslichkeit von mindestens 140 ml CO₂, insbesondere mindestens 180 ml CO₂, in 100 ml der Trägerflüssigkeit bei 37°C aufweisen. Zum Vergleich beträgt die Löslichkeit von CO₂ in arteriellem Blut bei 40 mmHg (etwa 5332,88 Pa) (physikalisch gelöst) etwa 2,6 ml/100 ml und bei 90 mmHg (11998,98 Pa) etwa 0,3 ml/100 ml.

Die Trägerflüssigkeit kann ein Perfluorcarbon oder eine Albumin- und/oder eine Elektrolytlösung, insbesondere angereichert mit spezifischen Proteinen oder Glukosederivaten, oder ein kommerziell erhältliches Dialysat, welches vorzugsweise zusätzlich über einen Ionenaustauscher, Aktivkohle oder einen anderen Adsorber aufbereitet wurde, sein.

Vorzugsweise kann die Erfindung mit einer Dialyseflüssigkeit als Trägermedium verwendet werden, welche entmineralisiertes Wasser, welches verschiedene Elektrolyte, sowie Bikarbonat in gleicher Konzentration enthält wie die physiologische extrazelluläre Flüssigkeit, umfasst oder daraus besteht. Im Betrieb können das Blut und die Dialyseflüssigkeit im Gegenstrom entlang einer semipermeablen Membran geführt werden, welche für niedrigmolekulare Substanzen (<5-15 kDa oder Kilodalton; bezogen auf die atomare Masseneinheit Dalton) durchlässig ist (Urämietoxine, Elektrolyten). Hierdurch kann ein maximaler Konzentrationsgradient nur für die überschüssigen Urämietoxine, nicht aber für die Elektrolyte, erzielt werden. Wenn zusätzlich Flüssigkeit abtrennt werden soll, kann ein Druckgradient erzeugt werden, welcher Lösung aus dem Blut abpresst. Auf diese Weise kann die Erfindung beipielsweise bei Schwermetallvergiftungen und Alkoholvergiftung angewendet werden.

Im Zusammenhang mit dem Austausch von CO₂ bzw. der Entfernung von CO₂ aus dem Blut hat sich als besonders vorteilhafte Trägerflüssigkeit ein Perfluorcarbon herausgestellt. D.h. die Trägerflüssigkeit ist vorzugsweise eine Flüssigkeit aus der Familie der Perfluorcarbone (PFC), welche unter physiologischen Bedingungen und bei Raumtemperatur flüssig ist. PFC weist eine zehnmal höhere Aufnahmekapazität für CO₂ als Blut auf (die Löslichkeit CO₂ in PFC beträgt 210 ml/dl, jene von O₂ 53 ml/dl). Abgesehen von Perfluorcarbonen kommen insbesondere andere Blutsubstitute, wie ein auf Hämoglobin basierter Sauerstoffträger (hemoglobin based oxygen carrier), in Frage. Die in Frage kommenden Perfluorcarbone weisen ferner eine niedrige Oberflächenspannung auf und breiten sich aufgrund ihres hohen Ausbreitungskoeffizienten sehr leicht auf Oberflächen aus. Diese Eigenschaften und ihr großen Potential zum Transport von CO₂ machen diese Flüssigkeiten besonders geeignet für den Einsatz zur Durchströmung einer Hohlfasermembran, um vor allem einen Teil des kontinuierlich gebildeten Kohlendioxids zur Entlastung der Atemarbeit während spontaner oder mechanisch unterstützter Beatmung unter niedrigen Blutflüssen zu entfernen. Die Hohlfasermembran wird daher vorzugsweise derart ausgelegt, dass die minimale Austauschoberfläche für einen effizienten CO₂-Transfer zur Verfügung steht, diese beträgt bei normalgewichtigen Patienten unter Blutflussraten von ca. 300 ml pro Minute (durch den Katheter / bei herkömmlichen Verfahren durch den Katheter - bei der Erfindung eigentlich nur Kontaktäquivalenzzeit) in der Größenordnung von mindestens 0,84 m².

Für den Austausch von protein-gebundenen Toxinen kann die Trägerflüssigkeit vorzugsweise Albumin umfassen. In diesem Fall kann die Erfindung zur Durchführung einer Albumindialyse verwendet werden. Dabei wird eine Membran verwendet, welche nur für die an das Albumin gebundenen Giftstoffe, sowie auch wasserlösliche kleine Molkeüle durchlässig ist. Das Konzentrationsgefälle von Giftstoffen ist auf der Blutkreislaufseite grösser als auf der Seite mit der albuminhaltigen Trägerflüssigkeit. Auf Grund des Konzentrationsgefälles und der Durchlässigkeit der Membran für die albumingebundenen Giftstoffe wandern diese durch die Membran (Diffusion) und werden von dem freien Albumin in der Trägerflüssigkeit gebunden. Durch einen Kreislauf kann das Albumin mit den Giftstoffen auf der Dialyseseite zu unterschiedlichen Entgiftungsstationen wandern. Beispielsweise kann als erste Entgiftungsstation eine komplette Dialyseapparatur, welche aber mehr die wassergelösten Moleküle (Giftstoffe) herausfiltert und den Elektrolytgehalt überprüft, vrogesehen sein. Als eine zweite Entgiftungsstation kann ein Aktivkohlefilter, der Giftstoffe filtert, die nicht anionisch sind, vorgesehen sein. Anionische Giftstoffe können den Kreislauf über einen Anionenfilter verlassen. Das so gereinigte Albumin kann dann wieder an der Membran Giftstoffe aufnehmen. Somit wird das Albumin auf der Blutkreislaufseite wieder frei und kann neue Giftstoffe transportieren. Das Blut wird entgiftet. Alternativ zu der beschriebenen Wiederaufbereitung kann die Trägerflüssigkeit mit dem Albumin auch nach einem Passieren der Membran verworfen werden.

Weiters können spezielle Trägerflüssigkeiten (z.B. Dialysate, physiologische Kochsalzlösungen) hergestellt oder gemischt werden, welche z.B. zur Behandlung von Elektrolytstörungen (Hyperkaliämie) oder zum Transport von Blutzucker, pathologischen Glucosemolekülen (zur Behandlung von Glykogenspeicherkrankheiten), Lipiden (zur Behandlung von Fettstoffwechselstörungen), Harnstoff oder Kreatinin, oder allgemein zur therapeutischen Apherese geeignet sind. Dabei können insbesondere speziell für den Austausch der jeweiligen Substanz(en) konfigurierte, z.B. semipermeable, Membranen zum Einsatz kommen. Besonders bevorzugte Trägerflüssigkeiten sind neben Perfluorcarbon insbesondere Albumin- und/oder Elektrolytlösungen oder kommerziell erhältliche Dialysate, welche zusätzlich über einem Ionenaustauscher, Aktivkohle oder einem anderen Adsorber aufbereitet wurden.

Die Erfindung kann beispielsweise als künstliche Pankreas verwendet werden, wobei das Blut durch die Membran Insulin aus der Trägerflüssigkeit aufnehmen und Glukagon an die Trägerflüssigkeit abgegeben kann. Die Membran kann beispielsweise aus Polyurethane hergestellt sein.

Bei Verwendung der oben genannten Trägerflüssigkeiten ermöglicht die Erfindung eine Vermeidung von Hyperkapnie, indem ein Teil des kontinuierlich gebildeten Kohlendioxids zur Entlastung der Atemarbeit während spontaner oder mechanischer Beatmung durch eine Flüssigkeit, insbesondere ein Perfluorcarbon entfernt wird. Perfluorcarbone weisen eine hohe Löslichkeit nicht für CO₂ sondern für alle respiratorischen Gase, daher auch für O₂, N₂ und NO, auf. Die Gasaustauschfläche der eingesetzten Membran braucht daher nicht so groß zu sein, wie es für herkömmliche intravaskuläre Membranoxygenatoren notwendig ist. Dadurch kann der Katheter in der Größe eines herkömmlichen Dialyse- oder Single Port ECMO-Katheters ausgeführt werden. Durch die Verwendung von flüssigem Perfluorcarbon, welches eine weitaus höhere Gasbindungskapazität aufweist als der bislang in herkömmlichen intravaskulären Membranoxygeneratoren verwendete Sauerstoff, ist zur Erzielung eines gleich guten Gasbindungseffektes eine viel geringere Kontaktfläche der Membran mit dem Blut notwendig, um eine effiziente Entfernung von CO₂ zu gewährleisten.

Die Erfindung kann außerdem mit einer Trägerflüssigkeit verwendet werden, welche Entkopplersubstanzen umfasst. Toxine können allgemein durch Substanzen verdrängt werden, die für die spezifischen Bindungsplätze am Protein oder am Lipid eine größere Affinität aufweisen als das Toxin. Diese Substanzen werden als Entkopplersubstanzen bezeichnet. Als Entkopplersubstanzen werden grundsätzlich physiologische Substanzen bevorzugt, jedoch können gegebenenfalls auch nichtphysiologische Substanzen, die als toxikologisch unbedenklich gelten, Verwendung finden. Diese Substanzen besitzen eine höhere spezifische Bindungsaffinität zu den Bindungsplätzen des Toxins, somit wird eine Verschiebung des chemischen Gleichgewichts bewirkt, und die Toxine werden freigesetzt. Die freigesetzten Toxine können dann aus dem Blut oder aus den Blutbestandteilen entfernt werden (enzymatisch, adsorptiv, Membranverfahren).

Zur Entfernung der freigesetzten Toxine aus der Trägerflüssigkeit kann bevorzugt ein Ultrafiltrationsverfahren angewendet. Hierbei können Lösungen, die aus verschiedenen Komponenten bestehen, nach Molekulargewicht getrennt werden. Dieses Verfahren eignet sich besonders zum Trennen von niedermolekularen Substanzen. Die Ausschlussgrenze, d.h. das Molekulargewicht der Stoffe, die zu ca. 90 % zurückgehalten werden, ist durch die gewählte Membran bestimmt. Die treibende Kraft ist eine Druckdifferenz zwischen den beiden Membranseiten. Das gewonnene Ultrafiltrat enthält primär die niedermolekularen Substanzen, d.h. im allgemeinen das Toxin. Es können jedoch auch geringe Mengen an Protein (ca. 1 %) im Filtrat enthalten sein. Bei der Ultrafiltration und Dialyse sind die Verfahrensweisen der einmaligen Passage und der Rezirkulation zu unterscheiden.

Hinsichtlich der Membran des Katheters ist es günstig, wenn die Membran eine selektiv permeable Membran ist, welche zumindest für die auszutauschende Substanz durchlässig ist. Je nach Anwendung, d.h. insbesondere in Abhängigkeit von der auszutauschenden Substanz und damit auch in Abhängigkeit von der Trägerflüssigkeit, kann der Aufbau, das Material und die Struktur der Membran entsprechend angepasst sein. Als Materialien kommen beispielsweise hydrophile oder hydrophilisierte Copolymere und hydrophile Polymermischungen in Frage. Im Einzelnen können Mischungen mit einer oder mehreren Komponenten einer Gruppe umfassend Polyethylen, thermoplastisches Polyurethan, Polysulfone (PSU), Polyethersulfone (PES), Polyacrylethersulfone (PAES), Polyvinylpyrrolidon (PVP), Polymethylmethacrylat (PMMA), Polyamid (PA), Polyacrylnitril (PAN) und/oder EthylenVinylalkohol-Copolymer (EVOH) sowie Cellulose, Cellulosetriacetat (CTA) oder Cellulosenitrat, als Membranmaterial verwendet werden. Bevorzugt sind Membranen im Wesentlichen nur aus PSU oder PES, im Wesentlichen aus einer Mischung aus PES, PVP und PA (PEPA) oder im Wesentlichen aus einer Mischung aus PAES, PVP und PA. Die Porengröße der Membran kann zwischen 0,01 um und 0,1 um liegen. Die Membran kann zusätzlich beschichtet sein, beispielsweise mit Heparin.

Eine besonders große Kontaktfläche auf kleinem Raum kann erzielt werden, wenn die Membran eine Hohlfasermembran ist. Die eigentliche Kontaktfläche wird dabei durch die Wände der Hohlfasern oder Kapillaren gebildet. Eine Hohlfasermembran kann bis zu 20.000 einzelne Kapillaren oder Hohlfasern umfassen. Der Durchmesser der einzelnen Hohlfasern beträgt zwischen 0,01 mm und 1 mm, insbesondere zwischen 0,1 und 0,5 mm. Die Gesamtoberfläche der Membran, welche die Kontaktfläche für den Austausch von Substanzen bildet, beträgt zwischen 0,01 und bis zu 10 m², vorzugsweise zwischen 0,1 und 1 m². Das Material aus dem dem Hohlfasern gebildet sind kann sich aus einer ober mehreren der oben genannten Komponenten zusammensetzen, wobei PMP (Polymethylpenten) bevorzugt zu nennen ist. Im Zusammenhang mit der erfindungsgemäßen Durchführung und gegebenenfalls Förderung des Bluts im Betrieb durch den Katheter können die filigranen Hohlfasern der Membran in einer schützenden Katheterhülle untergebracht sein. Der durch die große Kontaktfläche verursachte Strömungswiderstand kann durch die Fördereinrichtung zumindest teilweise kompensiert werden.

Weitere bevorzugte Membranmaterialien können Nanokapseln oder Mikrokapseln umfassen oder unter Verwendung solcher Kapseln hergestellt sein. Nanokapseln können durch Grenzflächenpolymerisation aus Polymeren wie Polyacrylaten, bevorzugt Poly(n-Butyl-Cyanacrylat) (PACA), sowie Poly(lactid-coglycolid) (PLGA), Albumin hergestellt werden. Solche Nanokapseln zeichnen sich durch eine sehr geringe Wandstärke von 3-20 nm aus. Sie können mit Perfluorocarbonen (PFC) gefüllt sein. Alternativ oder zusätzlich können den Nanokapseln bei der Herstellung auch Magnetpartikel sowie Fluoreszenzfarbstoffe (z.B. Nilrot) beigemischt werden, um sie besser spezifizieren und identifizieren zu können. (Delphine Moinard-Checot, Yves Chevalier, Stephanie Briancon et al. "Mechanism of nanocapsules formation by emuslion-diffusion process", Journal of Colloid and Interface Science 317 (2008) 458-468; Christian Erdmann, Christian Mayer, "Permeability profile of poly(alkyl canyoacrylate) nanocapsules", Journal of Colloid and Interface Science 478 (2016) 394-401)

Mikrokapseln können beispielsweise aus Silikon, bevorzugt UVhärtendem Silikon, z.B. Semicosil 949UV, durch eine Doppelkapillarmethode herstellen. Solche Mikrokapseln weisen eine Außenhülle aus Silikon und eine Füllung auf. Die Füllung kann Natriumkarbonat, Kaliumkarbonat, Magnesiumkarbonat, Natriumchlorid, physiologischer NaCl-Lsg, sowie Mischungen der genannten umfassen. Die Füllung kann weiters Carboanhydrase oder chemische Äquivalente hiervon wie Cyclen Zn (II) enthalten. Ebenso kann die Füllung aus in einem Trägermedium aufgenommenen PACA-Nanokapseln (wie oben definiert) bestehen. Weiters kann die Füllung der Mikrokapseln im Wesentlichen aus reinem Perfluorocarbonen oder Emulsionen aus PFC bestehen. Dem Kapselmaterial und/oder der Füllung können Fluoreszenzfarbstoffe beigemischt sein. Der Füllung können außerdem Farbindikatoren, welche bei ph-Wert Änderungen einen Farbumschlag zeigen (z.B. Thymol-blau) beigemischt sein, um die Sättigung der Substanzen nachverfolgen zu können. (A. S. Utada, E. Lorenceau, D. R. Link, P. D. Kaplan, H. A. Stone, D. A. Weitz, "Monodisperse Double Emulsions Generated from a Microcapillary Device", SCIENCE VOL 308 22 APRIL 2005; John J. Vericella, Sarah E. Baker,*, Joshuah K. Stolaroff et. al. "Encapsulated liquid sorbents for carbon dioxide capture", nature communications, 2015, DOI: 10.1038/ncomms7124)
Derartige Nanokapseln und/oder Mikrokapseln lassen sich beispielsweise mithilfe von Polymeren Cyanoacrylaten, Silikon (Loctite etc.) an die Oberfläche von Membranmaterialien wie Polymethylpenten (PMP), Polypropylen (PP) oder Silikon anbringen. Dabei entsteht eine chemische Bindung zwischen dem Trägermaterial und den Kapseln, welche zu einer Oberflächenvergrößerung beträgt und die Diffusionsgrenzschichte durch minimale Verwirbelungen aufbricht. Dementsprechend kann die Membran des vorliegenden Katheters mit Nanokapseln und/oder Mikrokapseln versehen sein.

Alternativ oder zusätzlich können derartige Nanokapseln und/oder Mikrokapseln können auch in bestehende Polymere wie Silikon eingebracht werden, indem sie z.B. eingerührt werden. Sie verteilen sich im Ausgangsmaterial und bilden beispielsweise mit PFC gefüllte Hohlräume, welche die Permeabilität des Ausgangsmateriales erhöhen. Alternativ lassen sich die Nanokapseln und/oder Mikrokapseln zwischen zwei dünne Polymerschichten, bevorzugt Silikon (Silpuran) einbringen. Hierbei werden Bubblefolien gebildet. Dies erhöht die Stabilität der Kapseln. Die so hergestellten Materialien eignen sich besonders zur Verwendung als Membran des vorliegenden Katheters.

Darüber hinaus können die Mikrokapseln und/oder Nanokapseln, beispielsweise mit Polymeren Cyanoacrylaten oder Silikon (Loctite etc.), miteinander verbunden werden und so zur Herstellung von Folien oder Hohlröhrchen verwendet werden, welche Folien oder Hohlröhrchen als Membran des vorliegenden Katheters verwendet werden können.

Die erfindungsgemäße Vorrichtung eignet sich besonders für minimalinvasive Anwendungen, beispielsweise in den Arm- und Beinvenen. Dabei orientieren sich die Abmessungen der erfindungsgemäßen Vorrichtung an den Abmessungen, die in der Kathetertechnologie verwendet werden (z.B. äußerer Durchmesser von 2,3 bis 12,7 mm oder 7 bis 40 Fr). Bevorzugt ist die Ausführung zur Applikation in Venen, wobei ein äußerer Durchmesser der Vorrichtung von 10 mm oder kleiner, vorzugsweise 8,7 mm oder kleiner, insbesondere 8,0 mm oder kleiner sich als praktisch besonders geeignet herausgestellt hat. Auch die Länge der erfindungsgemäßen Vorrichtung orientiert sich vorzugsweise an den gängigen Venenkatheter-Formaten, also etwa 100 bis 400 mm, insbesondere 150 bis 250 mm (im Blutgefäß). Ebenso gilt dies für die Materialien, die für die Außenseite verwendet werden; auch hier sollen bevorzugt die bereits aus der Kathetertechnologie bekannten Materialen verwendet werden.

Um den Strömungswiderstand und somit die zur vollen Kompensation erforderliche Leistung der Fördereinrichtung dennoch gering zu halten, ist es vorteilhaft, wenn die Fasern der Hohlfasermembran zu einem überwiegenden Teil im Wesentlichen parallel zu einer Längserstreckung des Katheters angeordnet sind. Die Längserstreckung des Katheters entspricht dabei naturgemäß der Hauptströmungsrichtung des Blutes in dem den Katheter im Betrieb umgebenden Gefäß.

Eine einfach Möglichkeit, den Zu- und Abfluss des Trägermediums am selben - vorzugsweise distalen - Ende des Katheters vorzusehen besteht darin, dass die Membran, insbesondere die Hohlfasermembran, gefaltet ist. Bei einer Faltung um 180° am gegenüber liegenden (proximalen) Ende des Katheters befinden sich beide Enden der Hohlfasern am selben Ende des Katheters.

Auch wenn grundsätzlich auch der Einsatz eines Einweg-Katheters mit einem - abgesehen von der Membran - geschlossenen Reservoir für das Trägermedium denkbar ist und die erfindungsgemäßen Vorteile ebenfalls genießt, ist es günstig, wenn der Katheter einen Einlass und einen Auslass für die Trägerflüssigkeit aufweist, welche mit einer extrakorporealen Austauscheinrichtung zur Bildung eines Kreislaufsystems mit der Austauscheinrichtung verbunden sind, wobei das Kreislaufsystem eine Pumpe zur Förderung der Trägerflüssigkeit aufweist. Es handelt sich in diesem Fall um einen Doppellumen-Katheter. Derartige Austauscheinrichtungen und Kreislaufsysteme sind grundsätzlich bekannt, wobei insbesondere auf Anwendungen zur Unterstützung der Lungenfunktion (ECMO, ECCO2R) und Dialyseanwendungen verwiesen wird.

Im Zusammenhang mit der Anwendung als Lungenunterstützung ist es günstig, wenn die Austauscheinrichtung ein Membranoxygenator ist. Es kann sich bei der Austauscheinrichtung aber auch um Absorber (zur Abscheidung von β₂-Mikroglobulin, Rheumafaktoren, Lipiden, Immunglobulinen oder Endotoxinen), z.B. Aktivkohle- oder Harz-Absorber, um Membran-Vorrichtungen zur Diffusion, Ultrafiltration und/oder Konvektion von Substanzen aus dem Trägermedium, oder um sonstige Filtervorrichtungen handeln.

Vorzugsweise kann eine solche Vorrichtung mit einem Katheter und einer extrakorporealen Austauscheinrichtung in einem Set zur Verfügung gestellt werden, wobei das Set zusätzlich zumindest einen mit dem Katheter und mit der Austauscheinrichtung verbundenen Schlauch zum Transport einer Trägerflüssigkeit zwischen dem Katheter und der Austauscheinrichtung umfasst. Der Schlauch weist dabei vorzugsweise zumindest zwei Kanäle oder Leitungen auf, wobei ein hineinführender Kanal zum Zuleiten der Trägerflüssigkeit zum Katheter und ein hinausführender Kanal zum Ableiten der Trägerflüssigkeit aus dem Katheter eingerichtet ist.

Eine besonders einfache kontinuierliche Anwendung wird ermöglicht, wenn bei dem Set die Austauscheinrichtung eine tragbare Austauscheinrichtung, vorzugsweise mit einer Trageeinrichtung, ist. Als Trageeinrichtung können beispielsweise Verbindungselemente für eine Trageschlaufe oder zur Befestigung an einem Gürtel oder einem sonstigen Kleidungsstück vorgesehen sein.

In Bezug auf das offenbarte Verfahren ist es besonders günstig, wenn die zur Diagnose zu entnehmende Substanz ein Krankheitsindikator ist, wovon auch körpereigene Substanzen, deren Vorhandensein (z.B. Antikörper) oder Quantität (z.B. Entzündungsproteine, Cytokine, Komplementfaktoren etc.) mit einer Erkrankung oder deren Verlauf korreliert, umfasst sind, insbesondere z.B. zumindest ein Krankheitserreger oder zumindest ein Antikörper, eine für den Körper toxische Substanz (z.B. Glucose oder Elektrolyte außerhalb des physiologischen Bereiches, wie Kalium, Kalzium etc.), eine durch den Körper sonst nicht ausscheidbare Substanz (vgl. Speichererkrankungen betreffend Glucose, Kupfer etc.), oder allgemein eine körpereigene Substanz deren Qualität oder Quantität mit einem Krankheitsverlauf korreliert, insbesondere zumindest ein krankheitsspezifisches Protein oder eine durch Krankheitsabläufe erzeugte Substanz (z.B. Komplement, Zytokine, Interleukine oder Antikörper). Wenn die Substanz beispielsweise Glucose ist, kann das Verfahren beispielsweise Teil eines Diagnoseverfahrens zur Blutzuckermessung sein kann.

Die Erfindung wird nachfolgend anhand von besonders bevorzugten Ausführungsbeispielen, auf die sie jedoch nicht beschränkt sein soll, und unter Bezugnahme auf die Zeichnungen noch weiter erläutert. Dabei zeigen im Einzelnen:
Fig. 1 schematisch einen Längsschnitt durch eine Vorrichtung mit einem intravaskulären Katheter mit einem seitlichen Bluteinlass und einer zentral angeordneten Hohlfasermembran;
Fig. 2 schematisch einen Längsschnitt durch eine Vorrichtung mit einem intravaskulären Katheter mit einem zentralen Blutdurchlass, seitlich angeordneten Hohlfasermembranen und einem proximalen Rückflusslumen;
Fig. 3 schematisch einen Querschnitt durch den Katheter entlang der Linie III-III in Fig. 2;
Fig. 4 schematisch einen Längsschnitt durch eine Vorrichtung mit einem intravaskulären Katheter mit einem zentralen Blutdurchlass und seitlich angeordneten Hohlfasermembranen ohne ein proximales Rückflusslumen;
Fig. 5 schematisch einen Längsschnitt durch eine Vorrichtung gemäß Fig. 4 mit einer motorgetriebenen Fördereinrichtung am distalen Ende des Blutdurchlasses;
Fig. 6 schematisch einen Längsschnitt durch eine Vorrichtung mit einem intravaskulären Katheter mit seitlich angeordneten Hohlfasermembranen, mit seitlichen Bluteinlässen und mit einer turbinengetriebenen Fördereinrichtung am distalen Ende des Katheters;
Fig. 7 eine weitere Ausführungsvariante eines intravaskulären Katheters mit einer tordierten Hohlfasermembran; und
Fig. 8 schematisch ein extrakorporales Kreislaufsystem zur Verwendung mit einem Katheter nach einer der Figuren 1 bis 7.

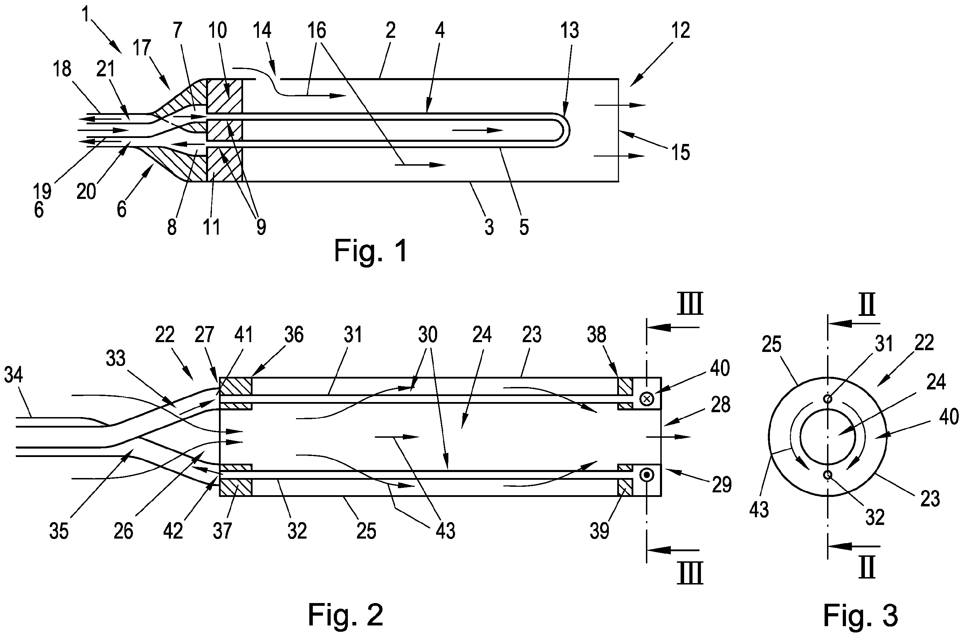

In Fig. 1 ist eine Vorrichtung 1 mit einem intravaskulären Katheter 2 im Längsschnitt schematisch dargestellt. Der Katheter 2 ist dafür vorgesehen, durch eine Vene eingeführt und in der unteren oder der oberen Hohlvene positioniert zu werden. Der Katheter 2 kann grundsätzlich von üblicher Ausführung sein und besitzt jene Eigenschaften, die für seine Verwendung bzw. Anwendung erforderlich sind. Der Katheter 2 umfasst einen Katheterschlauch 3. Der Katheterschlauch 3 weist einen im entspannten Zustand im Wesentlichen runden Querschnitt auf. Der Durchmesser des Katheterschlauchs 3 ist einer Vene angepasst, insbesondere kleiner als der Durchmesser einer Vene, in der der Katheter zum Einsatz kommen soll. Der Katheterschlauch 3 besteht aus einem für Katheter üblichen, elastischen Material, beispielsweise aus biokompatiblem Polyurethan. Im Katheterschlauch 3 ist eine längs des Katheterschlauch 3 verlaufende Membran 4', insbesondere eine Hohlfasermembran 4, angeordnet. Die Hohlfasermembran 4 ist der Einfachheit halber mit nur einer einzelnen Hohlfaser 5 dargestellt und umfasst in der Praxis eine Vielzahl von semipermeablen Hohlfasern aus einem der eingangs genannten bevorzugten Membranmaterialien. Wenn im Folgenden die Funktion der Hohlfaser 5 beschrieben ist, gelten die entsprechenden Ausführungen gleichermaßen für eine zweite und jede weitere Hohlfaser der Hohlfasermembran 4. Die Hohlfasermembran 4 ist derart ausgestaltet, dass am distalen Ende 6 des Katheters 2 über einen Einlass 7 für eine Trägerflüssigkeit die Trägerflüssigkeit in die Hohlfasermembran 4 eingeleitet werden kann, welche Trägerflüssigkeit die Hohlfaser 5 der Hohlfasermembran 4 durchströmt, und dass über einen Auslass 8 für die Trägerflüssigkeit die Trägerflüssigkeit abgeleitet werden kann. Die Trägerflüssigkeit durchströmt daher die Hohlfasermembran 4 bzw. deren Hohlfaser 5 zwischen dem Einlass 7 und dem Auslass 8. Die Innenseite der Hohlfaser 5 bildet somit eine erste Seite der Hohlfasermembran 4, welche mit der Trägerflüssigkeit in Kontakt steht. Die Enden 9 der Hohlfaser 5 sind in einem Verbindungsbereich 10 durch eine Einbettmasse 11 festgelegt und mit der Einbettmasse 11, z.B. einem Epoxidharz, verbunden. Im Bereich eines proximalen Endes 12 des Katheters weist die Hohlfaser 5 eine Beugung 13 auf, sodass die Hohlfaser 5 einen durchgehenden schlaufenförmigen Verlauf zwischen dem Einlass 7 und dem Auslass 8 aufweist.

Der intravaskuläre Katheter 2 ist ferner derart ausgeführt, dass er von Blut umspült und durchströmt werden kann. Zu diesem Zweck hat der Katheterschlauch 3 knapp außerhalb des Verbindungsbereichs 10 am distalen Ende 6 zumindest einen seitlichen Bluteinlass 14. Am proximalen Ende 12 ist der Katheterschlauch 3 geöffnet, sodass die Öffnung einen Blutauslass 15 bildet. Die Blutströmung im Katheter 2, in den Katheter 2 und aus dem Katheter 2 ist durch Richtungspfeile 16 angedeutet. Das Blut umströmt dabei ausgehend vom seitlichen Bluteinlass 14 die Hohlfaser 5 der Hohlfasermembran 4, sodass zumindest ein Teil des im Betrieb durch den Bluteinlass 14 in den Katheter 2 strömenden Bluts mit einer Außenseite der Hohlfaser 5 in Kontakt kommt, welche Außenseite eine der ersten Seite gegenüber liegende zweite Seite der Hohlfasermembran 4 bildet. Aufgrund des Materials der Hohlfaser 5 lässt diese und somit die Hohlfasermembran 4 insgesamt einen Austausch zumindest einer auszutauschenden Substanz zwischen der Trägerflüssigkeit im Inneren der Hohlfaser 5 und dem umgebenden Blut zu.

Der Einlass 7 und der Auslass 8 sind in einem Anschlussbereich 17 mit einem Zuführschlauch 18 verbunden. Der Zuführschlauch 18 weist einen koaxialen Innenschlauch 19 auf. In dem in Fig. 1 dargestellten Ausführungsbeispiel dient der im Inneren des Innenschlauchs 19 gebildete Kanal als Zulaufkanal 20 und der zwischen dem Innenschlauch 19 und dem Außenmantel des Zuführschlauchs 18 gebildete Kanal als Ablaufkanal 21 für die Trägerflüssigkeit. Der Zuführschlauch ist im Anschlussbereich 17 mittels einer elastischen Verbindungsmasse 22, z.B. Polyurethane, mit dem Katheter 2 verbunden.

In Fig. 2 und 3 ist ein weiteres Ausführungsbeispiel einer Vorrichtung 22 mit einem intravaskulären Katheter 23 mit einem zentralen Blutdurchlass 24 schematisch dargestellt. Der grundsätzliche Aufbau des Katheters 23 mit einem Katheterschlauch 25 entspricht dem in Zusammenhang mit Fig. 1 beschriebenen Katheter 2 und dem Katheterschlauch 3, sofern nicht nachfolgend anders beschrieben.

Der zentrale Blutdurchlass 24 verläuft als offener Kanal entlang einer Längsachse im Zentrum des Katheters 23. Der Blutdurchlass 24 verbindet somit einen zentralen Bluteinlass 26 am distalen Ende 27 des Katheters 23 und einen zentralen Blutauslass 28 am proximalen Ende 29 des Katheters 23 parallel zum Katheterschlauch 25.

Im Katheter 23 ist eine im Wesentlichen zylindrische Hohlfasermembran 30 angeordnet, wobei die semipermeablen Hohlfasern 31, 32 um den zentralen Blutdurchlass 24 herum und im Wesentlichen parallel zur Längserstreckung des Katheters 23 angeordnet sind. Die Hohlfasermembran 30 ist der Einfachheit halber mit nur zwei einzelnen Hohlfasern 31, 32 dargestellt und umfasst in der Praxis eine Vielzahl von semipermeablen Hohlfasern aus einem der eingangs genannten bevorzugten Membranmaterialien. Die erste Hohlfaser 31 ist am distalen Ende 27 des Katheters 23 mit einem Zulaufkanal 33 eines Zuführschlauchs 34 verbunden. Die zweite Hohlfaser 32 ist am distalen Ende 27 des Katheters 23 mit einem Ablaufkanal 35 des Zuführschlauchs 34 verbunden. Wenn im Folgenden die Funktion der ersten bzw. zweiten Hohlfaser 31, 32 beschrieben ist, gelten die entsprechenden Ausführungen gleichermaßen für je einen erste Teil aller weiteren Hohlfasern der Hohlfasermembran 30 entsprechend der ersten Hohlfaser 31 bzw. einen zweiten Teil aller weiteren Hohlfasern der Hohlfasermembran 30 entsprechend der zweiten Hohlfaser 32.

Entsprechend dem Katheter 2 in Fig. 1 sind die ersten Enden sämtlicher Hohlfasern 31, 32 in einem ersten ringförmigen Verbindungsbereich 36 am distalen Ende 27 des Katheters 23 durch eine Einbettmasse 37 festgelegt und mit der Einbettmasse 37, z.B. einem Epoxidharz, verbunden. Am proximalen Ende 29 des Katheters 23 sind die zweiten Enden sämtlicher Hohlfasern 31, 32 in einem zweiten ringförmigen Verbindungsbereich 38 ebenfalls durch eine Einbettmasse 39 festgelegt und mit der Einbettmasse 39, z.B. einem Epoxidharz, verbunden. Die Hohlfasern 31, 32 sind an ihren zweiten Enden an ein Rückflusslumen 40 angeschlossen, welches am distalen Ende 29 des Katheters 23 als ringförmiger Kanal innerhalb des Katheterschlauchs 25 gebildet ist. Die Hohlfasermembran 30 ist somit derart ausgestaltet, dass eine am distalen Ende 27 des Katheters 23 über einen Einlass 41 in die Hohlfasermembran 30 eingeleitete Trägerflüssigkeit die erste Hohlfaser 31 der Hohlfasermembran 30 durchströmt, am proximalen Ende 29 des Katheters in das Rückflusslumen 40 übertritt, zur zweiten Hohlfaser 32 geleitet wird, die zweite Hohlfaser 32 der Hohlfasermembran 30 durchströmt und über einen Auslass 42 abgeleitet wird.

Die Innenseite der Hohlfasern 31, 32 bilden somit eine erste Seite der Hohlfasermembran 30, welche mit der Trägerflüssigkeit in Kontakt steht. Die Blutströmung im Katheter 23, in den Katheter 23 und aus dem Katheter 23 ist durch Richtungspfeile 43 angedeutet. Das Blut umströmt dabei ausgehend vom zentralen Blutdurchlass 24 die Hohlfasern 31, 32 der Hohlfasermembran 30, sodass zumindest ein Teil des im Betrieb durch den Bluteinlass 26 in den Katheter 23 strömenden Bluts mit einer Außenseite der Hohlfasern 31, 32 in Kontakt kommt, welche Außenseite eine der ersten Seite gegenüber liegende zweite Seite der Hohlfasermembran 30 bildet. Aufgrund des Materials der Hohlfasern 31, 32 lassen diese und somit die Hohlfasermembran 30 insgesamt einen Austausch zumindest einer auszutauschenden Substanz zwischen der Trägerflüssigkeit im Inneren der Hohlfasern 31, 32 und dem umgebenden Blut zu.

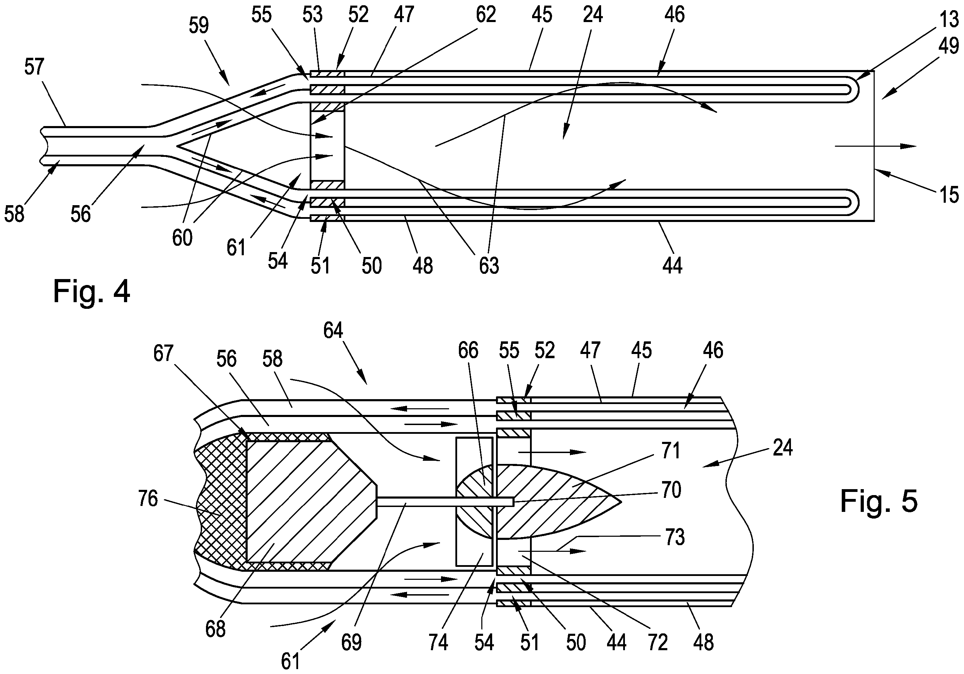

In Fig. 4 ist eine weitere alternative Ausführungsvariante der erfindungsgemäßen Vorrichtung mit einem Katheter 44 gezeigt. Der grundsätzliche Aufbau des Katheters 44 mit einem Katheterschlauch 45 entspricht wieder dem in Zusammenhang mit Fig. 1 bis 3 beschriebenen Katheter 2 bzw. 23, sofern nicht nachfolgend anders beschrieben.

Im Unterschied zu den vorangehend beschriebenen Kathetern 2, 23 ist gemäß Fig. 4 die Hohlfasermembran 46 des Katheters 44, welche die Membran 4' des Katheters bildet, zwar wie in Fig. 2 zylinderförmig um einen zentralen Blutdurchlass 24 angeordnet, jedoch sind die einzelnen Hohlfasern 47, 48 wie in Fig. 1 schlaufenförmig mit einer Beugung 13 im Bereich eines proximalen Endes 49 des Katheters 45 gebildet. Die Anordnung der Hohlfasermembran 46 entspricht somit einem auf halber Höhe nach außen gestülpten Zylinder. Die Enden 50, 51 der Hohlfasern 47, 48 sind in einem ringförmigen Verbindungsbereich 52 durch eine Einbettmasse 53 festgelegt und mit der Einbettmasse 53, z.B. einem Epoxidharz, verbunden. Die in Bezug auf eine zentrale Längsachse des Katheters 45 radial innen liegenden Enden 50 der Hohlfasern 47, 48 münden in einen ringförmigen Einlass 54 der Hohlfasermembran 46 für eine Trägerflüssigkeit. Die in Bezug auf eine zentrale Längsachse des Katheters 45 radial außen liegenden Enden 51 der Hohlfasern 47, 48 münden dementsprechend in einen ringförmigen Auslass 55 der Hohlfasermembran 46 für eine Trägerflüssigkeit, welcher konzentrisch dem Einlass 54 und radial außerhalb davon angeordnet ist. Der Einlass 54 der Hohlfasermembran 46 ist mit einem Zulaufkanal 56 eines Zuführschlauchs 57 verbunden. Der Auslass 55 der Hohlfasermembran 46 ist mit einem Ablaufkanal 58 des Zuführschlauchs 57 verbunden. Der Zuführschlauch 57 ist ansonsten gleich dem Zuführschlauch 18 gemäß Fig. 1 aufgebaut.

Die Kanäle 56, 58 des Zuführschlauchs 57 münden im dargestellten Beispiel an zwei radial gegenüberliegenden Stellen in den ringförmigen Einlass 54 bzw. den ringförmigen Auslass 55, sodass der Zuführschlauch 57 im Anschlussbereich 59 in zwei Schlauchäste 60 gegabelt ist. Die über den Einlass 54 in die Hohlfasermembran 46 eingeleitete Trägerflüssigkeit durchströmt die Hohlfasern 47, 48 der Hohlfasermembran 46 parallel zur Längserstreckung des Katheters 45 bis zur Beugung 13 der Hohlfasern 47, 48 und zurück zum distalen Ende 61 des Katheters und wird über den Auslass 55 abgeleitet.

Radial innerhalb des Einlasses 54 der Hohlfasermembran 46 weist der Katheter 45 am distalen Ende 61 einen zentralen Bluteinlass 62 in den Blutdurchlass 24 auf. Am proximalen Ende 49 ist der Katheterschlauch 44 geöffnet, sodass wie in Fig. 1 die Öffnung einen Blutauslass 15 bildet. Die Blutströmung im Katheter 45, in den Katheter 45 und aus dem Katheter 45 ist durch Richtungspfeile 63 angedeutet. Das Blut umströmt dabei ausgehend vom Bluteinlass 62 die Hohlfasern 47, 48 der Hohlfasermembran 46, sodass zumindest ein Teil des im Betrieb durch den Bluteinlass 62 in den Katheter 45 strömenden Bluts mit einer Außenseite der Hohlfasern 47, 48 in Kontakt kommt. Zur Vermeidung von Wiederholungen wird bezüglich des Austauschs von Substanzen mit dem Blut auf die entsprechenden Ausführungen zu Fig. 1 und 2 und den dort gezeigten Membranen verwiesen.

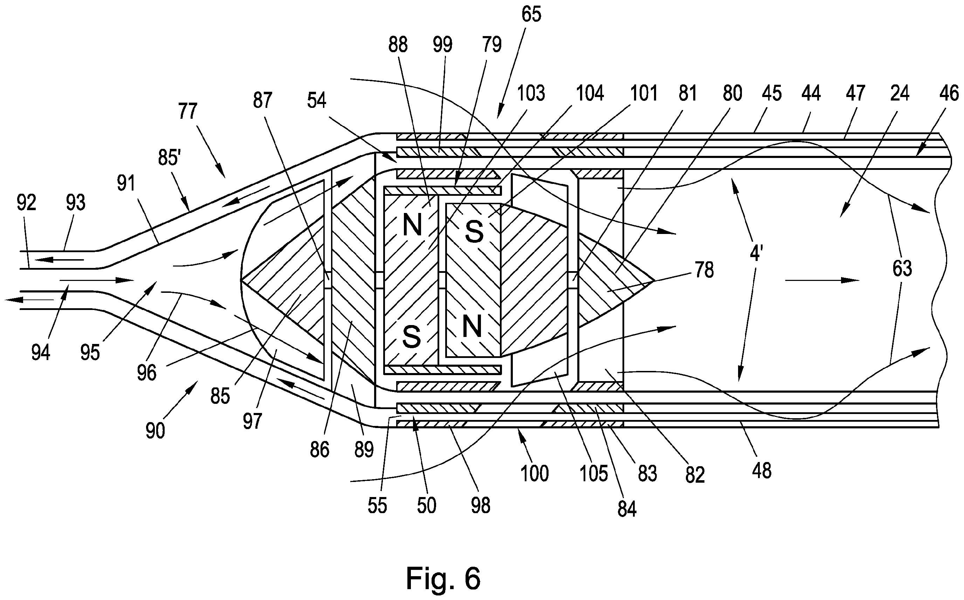

Nachdem die Vorrichtungen in Fig. 1 bis 4 auch der Einfachheit halber ohne Fördereinrichtungen gezeigt und beschrieben wurden, zeigen Fig. 5 und 6 jeweils eine Fördereinrichtung 64, 65, welche insbesondere in den in Fig. 2 und 4 gezeigten Kathetern 23 bzw. 44, vorzugsweise jeweils im Bluteinlass 26 bzw. 62 zum Einsatz kommen können. Dementsprechend sind die Katheter 44 in Fig. 5 und 6 nur andeutungsweise dargestellt und es wird bezüglich der Aufbaus und der Funktionsweise der Katheter 44 sowie der darin angeordneten Membranen auf die früheren Ausführungen im Zusammenhang mit den Figuren 1 bis 4 verwiesen.

Die in Fig. 5 dargestellte Fördereinrichtung umfasst einen Pumpenläufer 66 und eine Antriebseinheit 67 in Form eines Elektromotors 68. Der Elektromotor 68 überträgt im Betrieb über eine Welle 69 ein Drehmoment auf den Pumpenläufer 66. Die Welle 69 ist mit einem vom Elektromotor 68 gegenüber liegenden Ende 70 in einem Stator 71 gelagert. Der Stator 71 ist in einem Verbindungsbereich 52 über Flügel 72 im Katheter 44 befestigt. Die Flügel 72 sind dabei im Wesentlichen parallel oder leicht angewinkelt zu einer Strömungsrichtung (angedeutet durch die Richtungspfeile 73) des durch die seitlichen Bluteinlässe (nicht dargestellt) in den Katheter 44 eintretenden Bluts angeordnet. Der Pumpenläufer 66 weist seinerseits ebenfalls Flügel 74 auf, welche propellerartig zur axialen Beförderung des bei einer Rotation des Pumpenläufers 66 zwischen den Flügeln 74 befindlichen Bluts angeordnet sind.

Im Betrieb wird der Pumpenläufer 66 vom Elektromotor 68, welcher eine Antriebseinheit 85' bildet, derart angetrieben, dass eine Beschleunigung der Blutströmung im Bereich der Bluteinlässe und somit ein Überdruck am distalen Ende 61 des Katheters 44 erzeugt wird. Die Drehzahl des Elektromotors 68 wird dabei über eine Steuerung (nicht gezeigt) so gesteuert, dass der erzielte Überdruck eine Druckdifferenz zwischen den Bluteinlässen und dem Blutauslass 15 (vgl. Fig. 5) gerade ausgleicht. Dadurch wird effektiv der durch die Hohlfasern 47, 48 verursachte Strömungswiderstand im Inneren des Katheters 44 kompensiert. Die Menge des durch das Lumen des Katheters 44 bewegten Bluts entspricht somit derselben Menge, die durch den hohlen Katheterschlauch 45 bewegt werden würde, hätte der Katheter keine Membran.

Zur Festlegung gegenüber dem Stator 71 ist der Elektromotor 68 in einer Einbettmasse 76 eingebettet, welche den Elektromotor mit dem Katheterschlauch 44 verbindet.

Fig. 6 zeigt ein weiteres, bevorzugtes Ausführungsbeispiel für eine Fördereinrichtung 65. Die Fördereinrichtung 65 bildet das distale Ende 77 des Katheters 44. Die Fördereinrichtung 65 weist einen Pumpenläufer 78 auf, welcher zwischen einer Magnetkupplung 79 und einem Pumpenstator 80 drehbar angeordnet und mit einer Welle 81 im Pumpenstator 80 drehbar gelagert ist. Der Pumpenstator 80 ist über seitliche Flügel 82 in einem ersten Verbindungsring 83 befestigt. Der erste Verbindungsring 83 umfasst eine Einbettmasse 84, in der die Hohlfasern 47, 48 der Hohlfasermembran 46 eingebettet und damit verbunden sind, wobei die Hohlfasern 47, 48 den ersten Verbindungsring 83 axial, d.h. parallel zu einer Längsachse des Katheters 44 durchsetzen. Der erste Verbindungsring 83 ist an einer radialen Außenseite mit dem Katheterschlauch 45 des Katheters 44 verbunden.

Die Fördereinrichtung 65 weist als Antriebseinheit 85' weiters ein Turbinenelement 85 auf, welches in einem Turbinenstator 86 um eine Welle 87 drehbar gelagert ist. Die Welle 87 bildet eine drehfeste Verbindung des Turbinenelements 85 mit der Magnetkupplung 79, insbesondere mit einem antriebsseitigen Kupplungsteil 88. Der Turbinenstator 86 ist zwischen dem antriebsseitigen Kupplungsteil 88 und dem Turbinenelement 85, welches als Turbinenrotor fungiert, angeordnet, wobei die Welle 87 den Turbinenstator 86 durchsetzt. Der Turbinenstator 86 weist seitliche Flügel 89 auf, mit denen er in einem im Verbindungsbereich 90 erweiterten Abschnitt 91 des Innenschlauchs 92 eines Zuführschlauchs 93 befestigt ist. Das Turbinenelement 85 ist dementsprechend ebenfalls in dem erweiterten Abschnitt 91 angeordnet und somit der Strömung einer durch einen Zulaufkanal 94 des Zuführschlauchs 93 zugeführten Trägerflüssigkeit 95 ausgesetzt. Wie durch die Richtungspfeile 96 angedeutet, führt die Strömung der Trägerflüssigkeit 95 aus dem Zulaufkanal 94 in den erweiterten Abschnitt 91, über am Turbinenelement 85 zur Aufnahme eines Drehmoments angeordnete propellerartige Flügel 97 und vorbei an den Flügeln 89 des Turbinenstators 86 zum Einlass 54 der Hohlfasermembran 46.

Der ringförmige Einlass 54 und Auslass 55 der Hohlfasermembran 46 ist an einem zweiten Verbindungsring 98 gebildet, welcher - vergleichbar dem Verbindungsbereich 52 in Fig. 4 - eine Einbettmasse 99 umfasst, in der die Enden 50, 51 der Hohlfasern 47, 48 eingebettet sind, sodass sie in den Einlass 54 bzw. in den Auslass 55 münden. Zwischen dem zweiten Verbindungsring 98 und dem ersten Verbindungsring 83, welcher proximal des zweiten Verbindungsrings 98 angeordnet ist, weist der Katheter 44 seitliche Bluteinlässe 100 auf.

Die Magnetkupplung 79 umfasst neben dem antriebsseitigen Kupplungsteil 88 ein korrespondierendes abtriebsseitiges Kupplungsteil 101, welches mit dem Pumpenläufer 78 drehfest verbunden ist. Aufgrund der drehbaren Lagerung über die getrennten Wellen 81, 87 in den Statoren 80, 86 ist der antriebsseitige Kupplungsteil 88 relativ zu dem abtriebsseitigen Kupplungsteil 101 drehbar gelagert. Der abtriebsseitige Kupplungsteil 101 umfasst einen abtriebsseitigen zweipoligen Dauermagnet 102, welcher drehfest mit der Welle 81 des Pumpenläufers 78 verbunden ist. Der antriebsseitige Kupplungsteil 88 umfasst einen antriebsseitigen zweipoligen Dauermagnet 103, welcher drehfest mit der Welle 87 des Turbinenelements 85 verbunden ist. Der abtriebsseitige Dauermagnet 102 ist umfangseitig von einem im Wesentlichen topfförmigen Umleitelement 104 mit einem hohlzylinderförmigen Mantel umgeben. Dabei ist zwischen dem abtriebsseitigen Dauermagnet 102 und dem Umleitelement 104 ein Abstand bzw. Spalt vorgesehen, sodass der abtriebsseitige Kupplungsteil 101 berührungslos mit dem antriebsseitigen Kupplungsteil 88 gekoppelt ist. Das Umleitelement 104 besteht größtenteils aus ferromagnetischem Material. Der Mantel des Umleitelements 104 ist lediglich in einem schmalen Winkelbereich durch eine diamagnetische Trennung (nicht dargestellt) unterbrochen. Die Trennung teilt das Umleitelement 104 im Wesentlichen in zwei ferromagnetische Hälften bzw. Halbschalen. Eine durch die Trennung verlaufende Schnittebene steht somit senkrecht auf eine Magnetisierungsrichtung des mit dem Umleitelement 104 verbundenen antriebsseitigen zweipoligen Dauermagnets 103. Die durch die Trennung definierten ferromagnetischen Abschnitte des Umleitelements 104 sind daher entsprechend dem antriebsseitigen Dauermagnet 103 magnetisiert.

Aufgrund der berührungslosen Kopplung ist zwischen dem antriebsseitigen Kupplungsteil 88 und dem abtriebsseitigen Kupplungsteil 101 eine hermetische Trennung (nicht dargestellt) vorgesehen. Die hermetische Trennung ist durch eine mit der radialen Innenseite des zweiten Verbindungsrings 98 abdichtend verbundenen Folie gebildet.

Im Betrieb wird durch die Strömung der zugeführten Trägerflüssigkeit über die Flügel 97 ein Drehmoment auf das Turbinenelement 85 aufgebracht. Das Turbinenelement 85 überträgt das Drehmoment über die Welle 87 auf den antriebsseitigen Kupplungsteil 88 der Magnetkupplung 79. Durch die magnetischen Kräfte zwischen den Kupplungsteilen 88, 101 wird das Drehmoment von dem antriebsseitigen Kupplungsteil 88 auf das abtriebsseitige Kupplungsteil 101 übertragen, wobei die Stärke der magnetischen Kräfte ein bestimmtes maximal übertragbares Drehmoment definiert, jenseits dessen es zu einem "durchdrehen" der Kupplungsteile 88, 101 relativ zueinander kommt. Das abtriebsseitige Kupplungsteil 101 überträgt ein von dem antriebsseitigen Kupplungsteil 88 ausgeübtes Drehmoment über die Welle 81 auf den Pumpenläufer 78. Der Pumpenläufer 78 befördert mit seitlichen propellerartigen Flügeln 105 das zwischen den Flügeln 105 befindliche Blut von den Bluteinlässen 100 in Richtung des Blutdurchlasses 24 im Inneren des Katheters 44. Die Fördereinrichtung 65 erzeugt auf diese Weise eine Druckdifferenz zwischen den Bluteinlässen 100 und dem proximalen Ende des Blutdurchlasses 24, welche vorzugsweise eine Druckdifferenz zwischen dem proximalen und dem distalen Ende (nicht gezeigt) des Katheters 44 aufgrund des Strömungswiderstands der Hohlfasermembran 46 im Wesentlichen vollständig kompensiert. Das Turbinenelement 85 und der Pumpenläufer 78 sind dabei vorzugsweise so aufeinander abgestimmt, dass ein optimales Verhältnis zwischen der Strömungsgeschwindigkeit der Trägerflüssigkeit in den Hohlfasern 47, 48 und der Strömungsgeschwindigkeit des Bluts im Blutdurchlass 24 erzielt wird.

Die beiden Fördereinrichtungen 64, 65 sind in den obigen Ausführungsbeispielen jeweils im Bereich des distalen Endes 27, 77 des Katheters 44 angeordnet. Selbstverständlich sind auch Anordnungen an einer beliebigen Stelle im Katheter 44 denkbar, wodurch erwartungsgemäß ähnliche Vorteile erzielt werden können. Zudem sind selbstverständlich auch andere als die gezeigten Anordnungen der jeweiligen Rotoren (Pumpenläufer bzw. Turbinenelement) bezüglich der jeweiligen Statoren oder mit mehreren Statoren oder insgesamt nur einem Stator möglich, ohne die erfindungsgemäße Funktionsweise und damit den Bereich der Erfindung zu verlassen.

Darüber hinaus kann anstelle einer Hohlfasermembran auch ein anderer Membrantyp im Katheter zum Einsatz kommen, wobei der Fachmann die Fördereinrichtung 64, 65 an die zu erwartende Druckdifferenz aufgrund des unterschiedlichen Strömungswiderstands anderer Membrantypen anpassen wird.

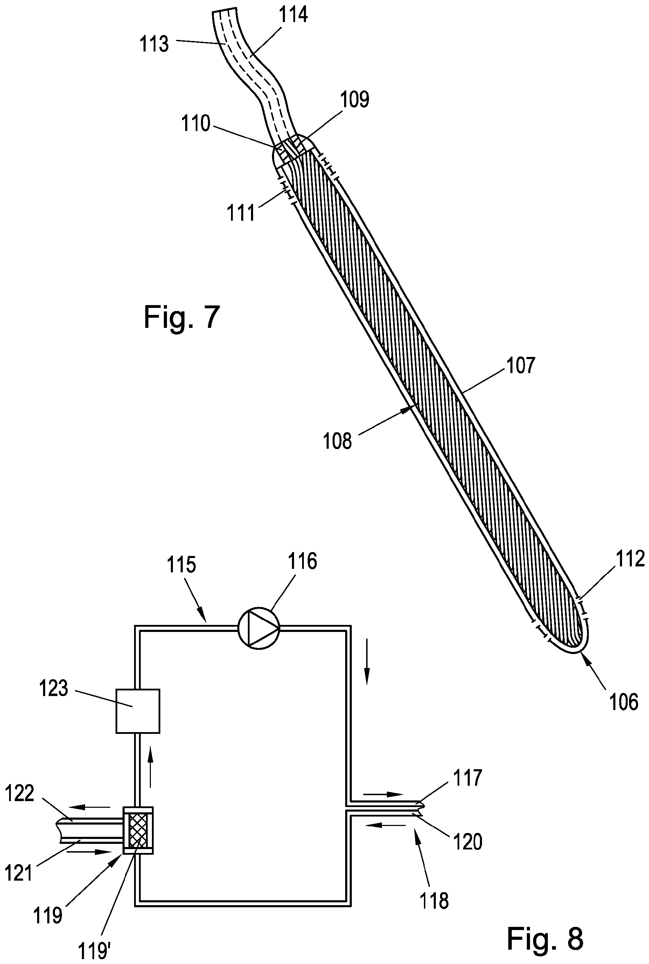

Fig. 7 zeigt eine weitere Ausführungsvariante eines intravaskulären Katheters 106, welcher dafür vorgesehen ist, durch eine Vene eingeführt und in der unteren oder der oberen Hohlvene positioniert zu werden. Der Katheter 106 kann grundsätzlich von üblicher Ausführung sein und besitzt jene Eigenschaften, die für seine Verwendung bzw. Anwendung erforderlich sind. Der Katheter 106 weist daher einen insbesondere mit rundem Querschnitt ausgeführten Katheterschlauch 107 auf, dessen Durchmesser an den Durchmesser der Vene angepasst ist, insbesondere geringfügig kleiner ist als der Durchmesser der Vene. Der Katheterschlauch 107 besteht aus einem für Katheter üblichen, elastischen Material, beispielsweise biokompatibles Polyurethan. Im Katheterschlauch 107 befindet sich ein längs des Schlauches 107 verlaufendes Hohlfasermembranmodul 108 mit einer Hohlfasermembran mit einer Vielzahl von gasdurchlässigen, jedoch flüssigkeitsundurchlässigen Hohlfasern aus einem der eingangs genannten Materialien, beispielsweise aus Polyethylen oder thermoplastischem Polyurethan. Das Hohlfasermembranmodul 108 ist derart ausgestaltet, dass am distalen Ende des Katheters 106 über einen ersten Katheteranschluss 109 in das Hohlfasermembranmodul 108 ein Medium, welches die Hohlfasern durchströmt, zugeleitet werden kann und über einen zweiten Katheteranschluss 110 das Medium abgeleitet werden kann. Das Medium, auf welches noch näher eingegangen wird, durchströmt daher das Hohlfasermembranmodul 108 zwischen dem ersten Katheteranschluss 109 und dem zweiten Katheteranschluss 110. Fig. 7 zeigt eine mögliche Ausführungsvariante des Hohlfasermembranmoduls 108 als Hohlfaserbündel, welches zwischen dem ersten Katheteranschluss 109 und dem zweiten Katheteranschluss 110 in einer Art Schlaufenform längs des Katheterinneren verläuft. Die einen Enden der Hohlfasern sind somit mit dem ersten Katheteranschluss 109 und die zweiten Enden mit dem zweiten Katheteranschluss 110 verbunden. An den Verbindungsbereichen können die Hohlfasern miteinander durch ein Epoxidharz oder dergleichen vergossen bzw. verbunden sein. Das schlaufenförmig verlaufende Hohlfaserbündel kann zusätzlich in sich verdreht bzw. tordiert sein. Der intravaskuläre Katheter 106 ist ferner derart ausgeführt, dass er von Blut umspült und durchströmt werden kann. Zu diesem Zweck kann der Schlauch 107 beispielsweise knapp außerhalb der beiden Katheteranschlüsse 109, 110 mit einer Anzahl von Einströmöffnungen 111 und im Bereich seines proximalen Endes mit einer Anzahl von Ausströmöffnungen 112 versehen sein.

Das Hohlfasermembranmodul 108 kann so ausgeführt sein, dass in einem Teil der Hohlfasern das Medium im Gleichstrom mit dem Blut und in einem anderen Teil der Hohlfasern das Medium im Gegenstrom zum Blut fließt. Die beiden Katheteranschlüsse 109, 110 sind bei der gezeigten Ausführungsform koaxial positioniert dargestellt, können jedoch auch, je nach Ausführung des Hohlfasermembranmoduls 108, nebeneinander angeordnet sein (vgl Fig. 1 oder Fig. 2). Die Katheteranschlüsse 109, 110 sind ferner mit flexiblen, über einen Abschnitt insbesondere koaxial verlaufenden Schläuchen, zu welchen ein mit dem ersten Anschluss 109 verbundener Zuführschlauch 113 und ein mit dem zweiten Anschluss 5 verbundener Ableitschlauch 114 gehören, verbunden.

Die bisher beschriebenen und in den Figuren 1 bis 7 gezeigten Katheter 2, 23, 44, 106 können Bestandteil einer größeren Vorrichtung sein, die mit einem der Katheter 2, 23, 44, 106 ein Kreislaufsystem 115 bildet, zu welchem weitere, extrakorporale Komponenten gehören. Fig. 8 zeigt schematisch eine Ausführungsform von solchen extrakorporal vorgesehenen Komponenten, nämlich eine Pumpe 116, welche eine Trägerflüssigkeit in den Zulaufkanal 117 eines Zuführschlauchs 118 und somit zum Katheter (in Fig. 8 nicht gezeigt) und durch diesen hindurch fördert. Die Pumpe 116 ist mit dem Zuführschlauch 118 verbunden. Eine weitere Komponente ist ein Oxygenator 119, in welchen die vom Ablaufkanal 120 kommende Trägerflüssigkeit eingeleitet wird. Der Oxygenator 119 kann ein herkömmlicher, handelsüblicher Membranoxygenator 119' mit einer Gaszuführung 121 und einer Gasabführung 122 sein. Im extrakorporalen Teil des Kreislaufes befinden sich ferner ein Wärmetauscher 123, welcher die Trägerflüssigkeit auf Körpertemperatur erwärmt, sowie weitere nicht dargestellte Komponenten, wie Druckmesseinrichtungen, Einrichtungen zum Messen des Durchflusses, Blasendetektoren und dgl..

Im Betrieb in einer Vene kommt ein Großteil des in der Vene transportierten, mit CO₂ angereicherten Blutes mit dem Hohlfasermembranmodul 108 zwischen dem distalen und dem proximalen Ende des Katheters 106 in Kontakt. Dabei wird die jeweils anwendungsspezifische Trägerflüssigkeit durch das Hohlfasermembranmodul 108 gepumpt, wobei die Trägerflüssigkeit das Hohlfasermembranmodul 108 zum Teil in Fließrichtung des Blutes und zum Teil gegen die Fließrichtung des Blutes durchströmt. An den Oberflächen der Hohlfasern geht beispielsweise Kohlendioxid (CO₂) aus dem Blut in die Trägerflüssigkeit über. Die mit CO₂ angereicherte Trägerflüssigkeit verlässt über den Ablaufkanal 120 das Hohlfasermembranmodul 108 sowie den Katheter 106 und wird in den externen Oxygenator 119 geleitet, wo das Kohlendioxid abgegeben und die Trägerflüssigkeit optional mit Sauerstoff angereichert wird. Der externe Oxygenator 119 wird bei einer einfachen Ausführungsform der Erfindung mit Umgebungsluft versorgt. Aus der Umgebungsluft nimmt die Flüssigkeit durch die Gasaustauschvorgänge im Oxygenator 119 auch Sauerstoff auf, sodass im Hohlfasermembranmodul 108 Sauerstoff an das durchströmende Blut abgegeben wird. Bei einer alternativen Ausführungsform der Erfindung kann die Flüssigkeit im Rahmen ihrer Sauerstoffkapazität durch Zuführung von Sauerstoff im Oxygenator 119 mit Sauerstoff angereichert werden. Die auf Körpertemperatur erwärmte Trägerflüssigkeit wird im Kreislauf wieder dem Hohlfasermembranmodul 108 zugeführt. Die Effizienz des Gastransfers im Hohlfasermembranmodul 108 ist dadurch, dass die Trägerflüssigkeit sowohl in Fließrichtung des Blutes als auch gegen die Fließrichtung des Blutes durch das Hohlfasermembranmodul 108 gepumpt wird, besonders hoch.

Analog zur Anwendung zum Austausch von CO₂ kann die Vorrichtung auch zur Entfernung anderer Substanzen, z.B. von Endotoxinen, aus dem Blut verwendet werden. Dabei kann als Trägerflüssigkeit ein entsprechend geeignetes flüssiges (z.B. kommerziell erhältliches) Dialysat oder dessen Aufbereitung durch Aktivkohle/Ionentauscher/Adsorber oder eine isotonische Flüssigkeit, angereichert mit Endotoxin neutralisierendem Proten (ENP) oder Albumin, vorgesehen werden. Anstelle eines geschlossenen Kreislaufsystems kann das Dialysat aus einem Tank entnommen, durch den Katheter gepumpt und anschließend in einem separaten Tank zur Entsorgung gesammelt werden.

Weiter alternativ kann in dem geschlossenen Kreislaufsystem 115 anstelle des Oxygenators 119 oder zusätzlich zum Oxygenator 119 eine Filtereinheit, z.B. mit einem Adsorptionsfilter, vorgesehen sein, sodass eine auszutauschende Substanz in der Filtereinheit vom Trägermedium abgetrennt wird.

Die erfindungsgemäße Vorrichtung ist als tragbare, kleine Einheit, die vom Patienten mitgeführt werden kann, ausführbar, insbesondere bei einer Ausführung, bei der dem externen Membranoxygenator 119 Umgebungsluft zugeführt wird. Die erfindungsgemäße Vorrichtung kann ferner in jedem herkömmlichen extrakorporalen Verfahren als Zusatzeinrichtung eingesetzt werden, vor allem auch in herkömmlichen Dialysekreisläufen.

## Patentansprüche

1. Vorrichtung umfassend einen Katheter (44) zur intravaskulären Verwendung, wobei der Katheter (44) einen Bluteinlass (100) und einen Blutauslass (15) aufweist und eine Membran (4') umfasst, wobei eine erste Seite der Membran (4') ein Lumen zur Aufnahme eines Trägermediums begrenzt und wobei die Membran (4') derart im Katheter (44) angeordnet ist, dass zumindest ein Teil des im Betrieb durch den Bluteinlass (100) in den Katheter (44) strömenden Bluts mit einer der ersten Seite gegenüber liegenden zweiten Seite der Membran (4') in Kontakt kommt bevor es den Katheter (44) durch den Blutauslass (15) verlässt, wobei die Membran (4') einen Austausch zumindest einer auszutauschenden Substanz zwischen einem im Betrieb in dem Lumen aufgenommenen Trägermedium und dem Blut zulässt, und wobei der Katheter (44) eine Fördereinrichtung (65) mit einer Antriebseinheit (85') zur Erzeugung eines Drehmoments und mit einem mit der Antriebseinheit (85') zur Übertragung eines Drehmoments verbundenen Pumpenläufer (78) umfasst, wobei die Fördereinrichtung (65) eingerichtet ist, im Betrieb eine Druckdifferenz zwischen dem Bluteinlass (100) und dem Blutauslass (15) zumindest teilweise zu kompensieren, und wobei die Membran (4') eine für Flüssigkeiten geeignete Membran ist, wobei das Trägermedium eine Trägerflüssigkeit ist, in welcher die auszutauschende Substanz löslich ist, und **dadurch gekennzeichnet, dass** der Pumpenläufer (78) über eine Magnetkupplung (79) mit der Antriebseinheit (85') verbunden ist, wobei die Magnetkupplung (79) zur Drehmomentübertragung entlang einer Drehachse zwei relativ zueinander drehbare Kupplungsteile (88, 101) mit jeweils einem Dauermagnet (103, 102) aufweist, wobei einer der Kupplungsteile (88) ein zumindest teilweise ferromagnetisches Umleitelement (104) umfasst, welches mit dem Dauermagnet (103) des Kupplungsteils (88) drehfest verbunden ist, wobei ein Teil des Umleitelements (104) radial außerhalb des Dauermagnets (102) des anderen Kupplungsteils (101) angeordnet ist, wobei das Umleitelement (104) zumindest eine diamagnetische Trennung aufweist, welche das Umleitelement (104) in zumindest zwei ferromagnetische Abschnitte unterteilt.

2. Vorrichtung nach Anspruch 1, **dadurch gekennzeichnet, dass** die Antriebseinheit (85') einen Elektromotor (68) umfasst.

3. Vorrichtung nach Anspruch 1, **dadurch gekennzeichnet, dass** die Antriebseinheit (85') ein Turbinenelement (85) umfasst, welches im Betrieb von dem Trägermedium umströmt wird.

4. Vorrichtung nach einem der Ansprüche 1 bis 3, **dadurch gekennzeichnet, dass** sie zur Verwendung mit Perfluorcarbon oder einer Albumin- und/oder einer Elektrolytlösung, insbesondere angereichert mit spezifischen Proteinen oder Glukosederivaten, oder eines kommerziell erhältlichen Dialysats, welches vorzugsweise zusätzlich über einen Ionenaustauscher, Aktivkohle oder einen anderen Adsorber aufbereitet wurde, als Trägerflüssigkeit eingerichtet ist.

5. Vorrichtung nach einem der Ansprüche 1 bis 4, **dadurch gekennzeichnet, dass** der Katheter (44) einen Einlass (54) und einen Auslass (55) für die Trägerflüssigkeit aufweist, welche mit einer extrakorporealen Austauscheinrichtung zur Bildung eines Kreislaufsystems (115) mit der Austauscheinrichtung verbunden sind, wobei das Kreislaufsystem (115) eine Pumpe (116) zur Förderung der Trägerflüssigkeit aufweist.

6. Set umfassend eine Vorrichtung nach Anspruch 5 und zumindest einen mit dem Katheter und mit der Austauscheinrichtung verbundenen Schlauch zum Transport einer Trägerflüssigkeit zwischen dem Katheter und der Austauscheinrichtung.

7. Set nach Anspruch 6, **dadurch gekennzeichnet, dass** die Austauscheinrichtung eine tragbare Austauscheinrichtung, vorzugsweise mit einer Trageeinrichtung, ist.

## Claims

1. Device comprising a catheter (44) for intravascular use, the catheter (44) having a blood inlet (100) and a blood outlet (15) and comprising a membrane (4'), a first side of the membrane (4') delimiting a lumen for receiving a carrier medium and the membrane (4') being arranged in the catheter (44) such that at least a portion of the blood flowing through the blood inlet (100) into the catheter (44) during operation comes into contact with a second side of the membrane (4'), opposite the first side, before the blood exits the catheter (44) through the blood outlet (15), the membrane (4') permitting an exchange of at least one substance to be exchanged between a carrier medium received in the lumen during operation and the blood, and the catheter (44) comprising a conveying device (65) having a drive unit (85') for generating a torque and having a pump rotor (78) connected to the drive unit (85') for transmitting a torque, the conveying device (65) being set up to at least partially compensate for a pressure difference between the blood inlet (100) and the blood outlet (15) during operation, and the membrane (4') being a membrane suitable for liquids, the carrier medium being a carrier liquid in which the substance to be exchanged is soluble, and **characterised in that** the pump rotor (78) is connected to the drive unit (85') by a magnetic coupling (79), the magnetic coupling (79) comprising two coupling parts (88, 101) which can be rotated relative to one another along a rotational axis and which each have a permanent magnet (103, 102), one of the coupling parts (88) comprising an at least partially ferromagnetic deflection element (104) which is connected to the permanent magnet (103) of the coupling part (88) for conjoint rotation therewith, part of the deflection element (104) being arranged radially outside the permanent magnet (102) of the other coupling part (101), the deflection element (104) having at least one diamagnetic separating means which divides the deflection element (104) into at least two ferromagnetic portions.

2. Device according to claim 1, **characterised in that** the drive unit (85') comprises an electric motor (68).

3. Device according to claim 1, **characterised in that** the drive unit (85') comprises a turbine element (85), around which the carrier medium flows during operation.

4. Device according to any of claims 1 to 3, **characterised in that** said device is set up to be used with perfluorocarbon or an albumin solution and/or an electrolyte solution, in particular is enriched with specific proteins or glucose derivatives, or a commercially obtainable dialysate, which has preferably been additionally processed by an ion exchanger, activated carbon or a different adsorber, as the carrier liquid.

5. Device according to any of claims 1 to 4, **characterised in that** the catheter (44) has an inlet (54) and an outlet (55) for the carrier liquid, which are connected to an extracorporeal exchange device in order to form a circulatory system (115) together with the exchange device, the circulatory system (115) having a pump (116) for conveying the carrier liquid.

6. Set comprising a device according to claim 5 and at least one hose connected to the catheter and to the exchange device for transporting a carrier liquid between the catheter and the exchange device.

7. Set according to claim 6, **characterised in that** the exchange device is a portable exchange device, preferably having a carrying device.

## Revendications

1. Dispositif comprenant un cathéter (44) pour l'utilisation intravasculaire, où le cathéter (44) présente une entrée de sang (100) et une sortie de sang (15) et comprend une membrane (4'), où un premier côté de la membrane (4') limite une lumière pour la réception d'un milieu vecteur et où la membrane (4') est agencée dans le cathéter (44) de telle manière qu'au moins une partie du sang s'écoulant pendant le fonctionnement par l'entrée de sang (100) dans le cathéter (44) vient en contact avec un second côté de la membrane (4') opposé au premier côté avant de quitter le cathéter (44) par la sortie de sang (15), où la membrane (4') permet un échange d'au moins une substance à échanger entre un milieu vecteur reçu pendant le fonctionnement dans la lumière et le sang, et où le cathéter (44) comprend un dispositif de transport (65) avec une unité d'entraînement (85') pour la production d'un moment de rotation et avec un rotor de pompe (78) relié à l'unité d'entraînement (85') pour la transmission d'un moment de rotation, où le dispositif de transport (65) est agencé pour compenser au moins en partie pendant le fonctionnement une différence de pression entre l'entrée de sang (100) et la sortie de sang (15), et où la membrane (4') est une membrane appropriée pour les liquides, où le milieu vecteur est un liquide vecteur dans lequel la substance à échanger est soluble, et **caractérisé en ce que** le rotor de pompe (78) est relié à l'unité d'entraînement (85') par le biais d'un accouplement magnétique (79), où l'accouplement magnétique (79) présente pour la transmission d'un moment de rotation le long d'un axe de rotation deux parties d'accouplement (88, 101) pouvant tourner l'une par rapport à l'autre avec dans chaque cas un aimant permanent (103, 102), où l'une des parties d'accouplement (88) comprend un élément de détournement (104) au moins en partie ferromagnétique, qui est relié de manière fixe en rotation à l'aimant permanent (103) de la partie d'accouplement (88), où une partie de l'élément de détournement (104) est agencé radialement à l'extérieur de l'aimant permanent (102) de l'autre partie d'accouplement (101), où l'élément de détournement (104) présente au moins une séparation diamagnétique qui divise l'élément de détournement (104) en au moins deux segments ferromagnétiques.

2. Dispositif selon la revendication 1, **caractérisé en ce que** l'unité d'entraînement (85') comprend un moteur électrique (68).

3. Dispositif selon la revendication 1, **caractérisé en ce que** l'unité d'entraînement (85') comprend un élément de turbine (85) qui pendant le fonctionnement est entouré par l'écoulement de milieu vecteur.

4. Dispositif selon l'une des revendications 1 à 3, **caractérisé en ce qu'**il est agencé pour l'utilisation avec un perfluorocarbure ou une solution d'albumine et/ou une solution d'électrolyte, en particulier enrichie avec des protéines ou des dérivés du glucose spécifiques, ou un dialysat disponible dans le commerce, qui de préférence a été préparé en outre par le biais d'un échangeur d'ions, de carbone activé ou d'un autre adsorbeur, comme liquide vecteur.

5. Dispositif selon l'une des revendications 1 à 4, **caractérisé en ce que** le cathéter (44) présente une entrée (54) et une sortie (55) pour le liquide vecteur, qui sont reliées à un dispositif d'échange extracorporel pour la formation d'un système à circulation (115) avec le dispositif d'échange, où le système à circulation (115) présente une pompe (116) pour le transport du liquide vecteur.

6. Ensemble comprenant un dispositif selon la revendication 5 et au moins un tuyau relié au cathéter et au dispositif d'échange pour le transport d'un liquide vecteur entre le cathéter et le dispositif d'échange.

7. Ensemble selon la revendication 6, **caractérisé en ce que** le dispositif d'échange est un dispositif d'échange portatif, de préférence avec un dispositif de manutention.
